(19) Europäisches Patentamt
European Patent Office
Office européen des brevets

(11) EP 1 216 039 B1

(12) EUROPÄISCHE PATENTSCHRIFT

(45) Veröffentlichungstag und Bekanntmachung des Hinweises auf die Patenterteilung:
**16.03.2005 Patentblatt 2005/11**

(21) Anmeldenummer: **00965957.4**

(22) Anmeldetag: **11.09.2000**

(51) Int Cl.[7]: **A61K 31/00**, A61P 15/10

(86) Internationale Anmeldenummer:
**PCT/EP2000/008836**

(87) Internationale Veröffentlichungsnummer:
**WO 2001/019357 (22.03.2001 Gazette 2001/12)**

(54) **KOMBINATIONPRÄPARAT ZUR BEHANDLUNG VON SEXUELLER DYSFUNKTION**
NOVEL COMBINATION FOR THE TREATMENT OF SEXUAL DYSFUNCTION
NOUVELLE COMBINAISON POUR LE TRAITEMENT D'UN DYSFONCTIONNEMENT SEXUEL

(84) Benannte Vertragsstaaten:
**DE ES FR GB IT**

(30) Priorität: **15.09.1999 DE 19944161**

(43) Veröffentlichungstag der Anmeldung:
**26.06.2002 Patentblatt 2002/26**

(73) Patentinhaber: **Bayer HealthCare AG**
**51368 Leverkusen (DE)**

(72) Erfinder:
- **BISCHOFF, Erwin**
  **42115 Wuppertal (DE)**
- **BISCHOFF, Hilmar**
  **42113 Wuppertal (DE)**
- **GIULIANO, François**
  **92210 Saint Cloud (FR)**

(56) Entgegenhaltungen:
**EP-A- 0 325 130** **WO-A-94/28902**
**WO-A-96/16644** **WO-A-99/24433**

- **M. D. CHEITLIN ET AL: "Use of Sildenafil (Viagra) in Patients With Cardiovascular Disease" CIRCULATION, Bd. 99, Nr. 1, 12. Januar 1999 (1999-01-12), Seiten 168-177, XP002164513**
- **WATANABE M ET AL: "SYNTHESIS AND BIOLOGICAL ANTIVITY OF METHANESULFONAMIDE PYRIMIDINE-AND N-METHANESULFONYL PYRROLE-SUBSTITUTED 3,5-DIHYDROXY-6-HEPTENOATES, A NOVEL SERIES OF HMG-COA REDUCTASE INHIBITORS" BIOORGANIC & MEDICINAL CHEMISTRY,GB,ELSEVIER SCIENCE LTD, Bd. 5, Nr. 2, 1997, Seiten 437-444, XP000882043 ISSN: 0968-0896 in der Anmeldung erwähnt**
- **BOOLELL M ET AL: "SILDENAFIL: AN ORALLY ACTIVE TYPE 5 CYCLIC GMP-SPECIFIC PHOSPHODIESTERASE INHIBITOR FOR THE TREATMENT OF PENILE ERECTILE DYSFUNCTION" INTERNATIONAL JOURNAL OF IMPOTENCE RESEARCH,STOCKTON, BASINGSTOKE,GB, Bd. 8, Nr. 2, Juni 1996 (1996-06), Seiten 47-52, XP000938747 ISSN: 0955-9930**
- **LAUFS U ET AL: "UPREGULATION OF ENDOTHELIAL NITRIC OXIDE SYNTHASE BY HMG COA REDUCTASE INHIBITORS" CIRCULATION,US,AMERICAN HEART ASSOCIATION, DALLAS, TX, Bd. 97, Nr. 12, 1998, Seiten 1129-1135, XP000870148 ISSN: 0009-7322 in der Anmeldung erwähnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 216 039 B1

Printed by Jouve, 75001 PARIS (FR)

## Beschreibung

Die vorliegende Erfindung betrifft das Gebiet der sexuellen Dysfunktion bei Männern und Frauen.

Insbesondere betrifft die vorliegende Erfindung ein neues Kombinationspräparat zur Behandlung der sexuellen Dysfunktion bei Männern und Frauen, insbesondere der erektilen Dysfunktion. Gegenstand der vorliegenden Erfindung ist somit auch eine neue Kombinationstherapie bei der Behandlung der sexuellen Dysfunktion bei Männern und Frauen.

Des Weiteren betrifft die vorliegenden Erfindung die Verwendung von Lipidsenkem zur Verstärkung der Wirkung von Phosphodiesterase-Hemmern (im folgenden synonym auch als "Phosphodiesterase-Inhibitoren", "PDE-Hemmer" oder "PDE-Inhibitoren" bezeichnet) bei der Behandlung der sexuellen Dysfunktion.

Aus dem Stand der Technik ist bekannt, dass Phosphodiesterase-Inhibitoren - insbesondere solche vom Subtyp V, welche synonym auch als "cGMP PDE-Inhibitoren" bezeichnet werden - sich für die Behandlung der sexuellen Dysfunktion eignen, und zwar insbesondere zur Behandlung der erektilen Dysfunktion (siehe z.B. *Molecular Pharmacology,* 1999, **56**, Seiten 124 - 130; *Am. J. Physiol.,* Vol. 264, Februar 1993, Seiten H419-H422; *The Journal of Urology,* Vol. 147, Seiten 1650-1655 (Juni 1992); *The New England Journal of Medicine,* Vol. 326(2), Seiten 90-94 (9. Januar 1992); *International Journal of Impotence Research,* **4**, Suppl. 2, Seite 11 (1992); *Drugs, News and Perspectives,* 6(3), Seiten 150-156 (April 1993); *Physiological Reviews* **75,** Seiten 191-236 (1995); *Int. J. of Impotence* **9**, Seiten 17-26 (1997) und *TIPS Reviews,* Vol. 11, Seiten 150-155 (April 1990)).

Zur Nomenklatur der PDE-Inhibitoren wird auf *Beavo* und *Reifsnyder* in *Trends in Pharmacol. Sci. 1990.* ***11, Seiten 150-155*** und auf den Artikel *TIPS Reviews,* Vol. 11, Seiten 150-155 (April 1990) verwiesen.

Der Grund für die Wirksamkeit von PDE-Hemmern, insbesondere cGMP PDE-Hemmem (PDE V-Hemmem), bei der Behandlung der sexuellen, vorzugsweise erektilen Dysfunktion liegt darin, dass der durch sexuelle Stimulation vom Körper generierte Neurotransmitter Stickstoffmonoxid NO die Guanylatcyclase aktiviert, die ihrerseits GTP zu cGMP umwandelt, welches dann seinerseits eine Relaxation des Corpus cavemosum bewirken kann, ohne dass das für die Relaxation des Corpus cavemosum verantwortliche cGMP vom Enzym Phosphodiesterase V (PDE V) zum 5'GMP hydrolysiert wird, weil die Aktivität des Enzyms PDE V durch den entsprechenden Inhibitor gehemmt wird. Dieser Mechanismus ist in der beiliegenden **Fig. 1** veranschaulicht.

Man hat aber nun festgestellt, dass eine Therapie der erektilen Dysfunktion mit PDE-Inhibitoren, insbesondere cGMP PDE-Inhibitoren, bei einigen Patienten nicht oder nur bedingt anspricht. Bei dieser Patientengruppe handelt es sich insbesondere um Patienten mit gestörter Endothelfunktion und/oder Stoffwechselerkrankungen, wie z.B. Hyperlipidämie (z.B. Hypercholesterinämie), Arteriosklerose, Diabetes (insbesondere vom Typ Diabetes mellitus), aber auch um starke Raucher und ältere Patienten. Bei diesen Patienten beobachtet man bei der Therapierung der erektilen Dysfunktion mit den üblichen Dosen nur einen deutlichen verminderten Effekt im Vergleich zu anderen Patientengruppen, bei denen zwar eine erektile Dysfunktion therapiert wird, die aber nicht an den zuvor genannten Stoffwechselerkrankungen leiden.

Nun leidet aber gerade die zuvor genannte Patientengruppe mit gestörter Endothelfunktion und/oder den zuvor genannten Stoffwechselerkrankungen überdurchschnittlich häufig an sexueller Dysfunktion, insbesondere erektiler Dysfunktion, wodurch die herkömmliche Behandlung dieser Dysfunktion mit PDE-Hemmern erschwert wird. Bei diesen Problempatienten müssen - im Vergleich zu anderen Patientengruppen mit erektiler Dysfunktion, jedoch ohne die zuvor genannten Stoffwechselerkrankungen - deutlich höhere Dosen an PDE-Inhibitoren verabreicht werden, um einen therapeutischen Effekt zu erzielen. Dies bringt aber - neben Kostengesichtspunkten - auch den wesentlichen Nachteil mit sich, dass auch die mit der PDE-Inhibitor-Therapie einhergehenden Nebenwirkungen um den Faktor der Höherdosierung zunehmen. Zu diesen Nebenwirkungen zählen beispielsweise eine Beeinträchtigung des Sehvermögens, insbesondere gestörtes Farbsehen und Farbempfinden, Kopfschmerzen sowie Muskelschmerzen. Es besteht auch die Möglichkeit von Nebenwirkungen auf das Herzkreislaufsystem z.B. eine Senkung des Blutdruckes.

Die Anmelderin hat nun überraschenderweise herausgefunden, dass die Wirkung von PDE-Inhibitoren, insbesondere PDE V-Inhibitoren, bei der Therapie der sexuellen, vorzugsweise erektilen Dysfunktion - insbesondere bei den zuvor genannten Problempatienten-Gruppen - verstärkt werden kann, wenn in Kombination zu den PDE-Inhibitoren ein Lipidsenker verabreicht wird. Auf diese Weise können die zuvor geschilderten Nachteile, die bei der herkömmlichen Behandlung der erektilen Dysfunktion nur mit PDE-Inhibitoren allein insbesondere bei den zuvor erwähnten Problempatienten auftreten, vermieden werden.

Somit betrifft die vorliegende Erfindung eine Kombinationspräparat, enthaltend

- als Wirkstoffkomponente A mindestens einen PDE V-Inhibitor (cGMP PDE-Inhibitor); und
- als Wirkstoffkomponente B mindestens einen Lipidsenker.

Das erfindungsgemäße Kombinationspräparat ist insbesondere für die Therapie der sexuellen Dysfunktion geeignet, d.h. die Therapie der erektilen Dysfunktion bei Männern oder der sexuellen Dysfunktion bei Frauen.

Gleichzeitig kann mit dem erfindungsgemäßen Kombinationspräparat auch eine gestörte Endothelfunktion (z.B. bei älteren Patienten oder starken Rauchern) und/oder eine Stoffwechselerkrankung mittherapiert werden, so z. B. Hyperlipidämie (z.B. Hypercholesterinämie), Arteriosklerose oder Diabetes (insbesondere vom Typ Diabetes mellitus).

Der Begriff "Kombinationspräparat", wie er im Sinne der vorliegenden Erfindung verwendet wird, bedeutet, dass die beiden Wirkstoffkomponenten A und B entweder gleichzeitig oder aber auch zeitlich abgestuft (d.h. also getrennt voneinander) angewandt werden können.

Somit umfasst der Begriff "Kombinationspräparat" erfindungsgemäß die Bestandteile A und B entweder in einer funktionellen Einheit, d.h. als echte Kombination (z.B. als Mischung, Gemisch oder Gemenge), oder aber auch (räumlich) getrennt nebeneinander, d.h. als sogenanntes "kit-of parts".

Gegenstand der vorliegenden Erfindung ist somit auch die Verwendung von Lipidsenkem zur Steigerung der Wirksamkeit von PDE V-Inhibitoren bei der Therapie der sexuellen Dysfunktion, insbesondere der erektilen Dysfunktion.

Weiterer Gegenstand der vorliegenden Erfindung ist somit auch eine Kombinationstherapie für die sexuelle Dysfunktion, insbesondere für die erektile Dysfunktion, mit einem Kombinationspräparat, das mindestens einen PDE V-Inhibitor und mindestens einen Lipidsenkem umfasst.

Gleichzeitig kann durch die erfindungsgemäße Kombinationstherapie auch eine gestörte Endothelfunktion (z.B. bei älteren Patienten oder starken Rauchern) und/oder eine Stoffwechselerkrankung mittherapiert werden, so z. B. Hyperlipidämie (z.B. Hypercholesterinämie), Arteriosklerose oder Diabetes (insbesondere vom Typ Diabetes mellitus).

Durch die erfindungsgemäße Kombination von PDEV-Hemmer und Lipidsenker - d.h. also mit anderen Worten durch die erfindungsgemäße Kombinationstherapie der sexuellen Dysfunktion mit einer Kombination von PDEV-Hemmer und Lipidsenker - lassen sich die für die Therapie erforderlichen Dosen an PDEV-Hemmern bei den oben genannten Problempatienten wieder auf übliche Dosen zurückführen, wie sie auch bei anderen Patienten, welche zwar an erektiler Dysfunktion, aber nicht an gestörter Endothelfunktion oder einer Stoffwechselerkrankung leiden, verabreicht werden. Aber auch bei Patienten ohne spezifische Stoffwechselstörung, die aber (z.B. altersbedingt) an erektiler Dysfunktion leiden, kann erfindungsgemäß die zur Therapie der erektilen Dysfunktion verabreichte Dosis an PDEV-Hemmer durch die kombinierte Lipidsenkergabe reduziert werden.

Wie zuvor erwähnt, kann die erfindungsgemäße Kombination so verabreicht werden, d.h. die erfindungsgemäße Kombinationstherapie dadurch erfolgen, dass die Wirkstoffkomponenten A und B gleichzeitig verabreicht werden. Dabei können in die Wirkstoffkomponenten A und B, wie zuvor geschildert, entweder in einer funktionellen Einheit (d.h. als echte Kombination wie z.B. als Mischung, Gemisch oder Gemenge) oder aber auch (räumlich) getrennt nebeneinander (d.h. als sogenanntes "kit" oder "kit-of parts") vorliegen.

Gemäß einer bevorzugten Ausführungsform der vorliegenden Erfindung werden die Wirkstoffkomponenten A und B getrennt voneinander verabreicht, und zwar insbesondere zeitlich abgestuft.

Dies kann beispielsweise dadurch geschehen, dass einige Tage (z.B. etwa 1 Woche oder auch nur 1-4 Tage) vor Verabreichung des PDEV-Hemmers bereits eine tägliche Dosis des Lipidsenkers verabreicht wird.

Auch besteht die Möglichkeit, den PDEV-Hemmer in eine bereits bestehende Lipidsenker-Therapie hinein zu verabreichen. So lassen sich beispielsweise gemäß der vorliegenden Erfindung verbesserte Therapieerfolge der erektilen Dysfunktion mit PDE-Hemmern bei Männern mit starker Hypercholesterinämie beobachten, bei denen die erhöhten Cholesterinspiegel bereits dauerhaft mit Lipidsenkem behandelt werden.

Ähnlich gute Therapieerfolge werden überraschenderweise aber auch in solchen Fällen beobachtet, bei denen der Lipidsenker erst kurzfristig (z.B. nur wenige Tage) vor Gabe des PDEV-Hemmers verabreicht wird. Dies ist überraschend und weist auf einen nicht vorhersehbaren synergistischen Effekt der erfindungsgemäßen Kombination hin, weil bei den so behandelten Patienten, die z.B. an Arteriosklerose leiden, aufgrund der kurzen Therapiedauer mit dem Lipidsenker überhaupt noch keine Änderung ihres Krankheitsbildes eingetreten sein kann.

Gemäß einer bevorzugten Ausführungsform der vorliegenden Erfindung werden die Wirkstoffbestandteile A und B des erfindungsgemäßen Kombinationspräparates also zeitlich abgestuft verabreicht, vorzugsweise der Lipidsenker vorab, d.h. zeitlich vor Gabe des PDEV-Hemmers. Dies kann dadurch geschehen, dass der Lipidsenker einfach kurz vor Gabe des PDEV-Hemmer, d.h. mehrere Male über einige Tage verteilt oder aber auch nur einmal wenige Stunden zuvor, verabreicht wird, oder aber auch dadurch, dass der PDEV-Hemmer in eine bereits bestehende Therapie mit einem Lipidsenker hinein verabreicht wird. Im letzteren Fall kann die Gabe des Lipidsenkers also auch vor und parallel zur Gabe des PDEV-Hemmers fortgesetzt werden.

Ohne sich hierbei auf eine bestimmte Theorie festlegen zu wollen, lässt sich die Verbesserung der PDE-inhibitorischen Wirkung des PDE-Hemmers durch die gleichzeitige oder zeitlich abgestufte oder parallele Gabe von Lipidsenkem vermutlich dadurch erklären, dass die Lipidsenker die gestörte Endothel-Funktion durch Generierung von Stickstoffmonoxid (NO) verbessern *(Current Opinion in Lipidology,* 1997, Vol. 8, Seiten 362-368 und *Circulation* 1998, **97,** Seiten 1129-1135). Stickstoffmonoxid als Neurotransmitter wiederum ist ein wichtiger physiologischer Faktor für

die Ausbildung einer Erektion, da es zur Erhöhung des cGMP Spiegels und schließlich zur Relaxation des Corpus cavemosum führt *(International Journal of Impotence Research* 1999, **11,** Seiten 123 - 132 und 159 - 165).

Gemäß der vorliegenden Erfindung kann der Lipidsenker ausgewählt sein der Gruppe von:

- HMG-CoA-Reduktase-Inhibitoren,
- Squalen-Synthase-Inhibitoren,
- Gallensäure-Absorptionshemmern (auch "Gallensäure-Anionenaustauscher" oder "Bile acid sequestrants" genannt),
- Fibrinsäure und ihren Derivaten,
- Nikotinsäure und ihren Analogen sowie
- ω3-Fettsäuren.

Für weitere Einzelheiten zu den zuvor genannten Lipidsenkern wird in diesem Zusammenhang verwiesen auf den Aufsatz von Gilbert R. Thompson & Rissitaza P. Naoumova "New prospects for lipid-lowering drugs" in *Exp. Opin. Invest. Drugs* (1998), **7**(5), Seiten 715 - 727.

Unter den zuvor genannten Lipidsenkem werden die HMG-CoA-Reduktase-Inhibitoren erfindungsgemäß bevorzugt. Die Abkürzung "HMG-CoA" steht hierbei für "3-Hydroxymethylglutaryl-Coenzym A".

Unter den HMG-CoA-Reduktase-Inhibitoren wiederum wird erfindungsgemäß insbesondere die Substanzklasse der Vastatine - der Einfachheit halber in der Literatur meist nur als "Statine" bezeichnet - bevorzugt.

Unter den Statinen wiederum erfindungsgemäß besonders bevorzugt sind

- Atorvastatin (im Handel erhältlich unter der Bezeichnung Lipitor® von Parke-Davis);
- Cerivastatin (im Handel erhältlich unter der Bezeichnung Lipobay® oder Baycol® von Bayer);
- Fluvastatin (im Handel erhältlich unter der Bezeichnung Lescol® von Novartis);
- Lovastatin (im Handel erhältlich unter der Bezeichnung Mevacor® von Merck);
- Pravastatin (im Handel erhältlich unter der Bezeichnung Lipostat® von Bristol-Myers Squibb);
- Simvastatin (im Handel erhältlich unter der Bezeichnung Zocor® von Merck);
- Itavastatin (auch "Nisvastatin" genannt; NK-104; systematischer Name: [S-[R*,S*-(E)]]-7-[2-Cyclopropyl-4-(4-fluorphenyl)-3-chinolinyl]-3,5-dihydroxy-6-heptensäure);
- Dalvastatin;
- Mevastatin;
- Dihydrocompactin;
- Compactin; und
- (+)-(3R,SS)-Bis-(7-(4-(4-fluorphenyl)-6-isopropyl-2-(N-methyl-N-methansulfonylamino)-pyrimidin-S-yl)-3,S-dihydroxy-6(E)-heptensäure;

sowie deren jeweilige Salze, Hydrate, Alkoholate, Ester und Tautomere,
hierunter ganz besonders bevorzugt Atorvastatin, Cerivastatin, Fluvastatin, Lovastatin, Pravastatin, Itavastatin, Simvastatin und (+)-(3R,5S)-Bis-(7-(4-(4-fluorphenyl)-6-isopropyl-2-(N-methyl-N-methansulfonylamino)-pyrimidin-5-yl)-3,5-dihydroxy-6(E)-heptensäure sowie deren jeweilige Salze, Hydrate, Alkoholate, Ester und Tautomere.

Hierunter wiederum ganz besonders bevorzugt sind das Cerivastatin und das Atorvastatin sowie deren jeweilige Salze, Hydrate, Alkoholate, Ester und Tautomere.

Für weitere Einzelheiten zu den zuvor genannten Statinen wird verwiesen auf die Abhandlungen in *Drugs of the Future* 1994, **19**(6), Seiten 537 - 541 sowie 1995, **20**(6), Seite 611 sowie 1996, 21(6), Seite 642.

Der Begriff "Salz" im Sinne der vorliegenden Erfindung meint jeweils physiologisch unbedenkliche Salze der jeweiligen Verbindungen: Dies können z.B. können Salze mit Mineralsäuren, Carbonsäuren oder Sulfonsäuren sein, insbesondere mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure oder auch Mischsalze hiervon. Es kann sich aber auch um Salze mit üblichen Basen handeln, wie beispielsweise Alkalimetallsalze (z.B. Natriumoder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder Methyl-piperidin sowie Mischsalze hiervon.

Beispiele für erfindungsgemäß verwendbare Statin-Salze sind das Fluindostatin (das Mononatriumsalz des Fluvastatins); das Monokaliumsalz und das Calciumsalz des Itavastatins; sowie das Calciumsalz der (+)-(3R,5S) -Bis-(7-(4-(4-fluorphenyl)-6-isopropyl-2-(N-methyl-N-methane-sulfonylamino)-pyrimidin-5-yl)-3,5-dihydroxy-6(E)-heptensäure ("ZD 4522" oder "S 4522" von den Firmen Shionogi bzw. AstraZeneca). Weitere Beispiele für erfindungsgemäß verwendbare Statinsalze sind die Mononatrium- und die Monokaliumsalze sowie die Calciumsalze des Cerivastatins, des Atorvastatins und des Pravastatins.

Weitere bevorzugte HMG-CoA-Reduktase-Inhibitoren sind beschrieben in der EP-A-0 325 130 und in der EP-A-0-491 226, beide im Namen der Bayer AG. Gegenstand der EP-A-0 325 130 sind substituierte Pyridine, und in der EP-A-0-491 226 sind substituierte Pyridyldihydroxyheptensäurederivate und ihre Salze beschrieben, hierunter insbesondere das erfindungsgemäß besonders bevorzugte Cerivastatin (Anspruch 6 der EP-A-0-491 226).

Erfindungsgemäß ebenfalls bevorzugt sind die in der WO-A-99/11263 genannten Statine,

Erfindungsgemäß gleichemiaßen bevorzugt sind die HMG-CoA-Reduktase-Inhibitoren, welche in der Druckschrift *Bioorganic & Medicinal Chemistry,* Vol. 5, No. 2, Seiten 437-444 (1997) genannt sind.

Eine weitere Übersicht über HMG-CoA-Reduktase-Hemmer ist in *Pharmazie in unserer Zeit,* 28. Jahrg., Nr. 3, Seiten 147-1152 (1999) enthalten.

Unter den zuvor genannten Gallensäureabsorptionshemmern ("Bile acid sequestrants") erfindungsgemäß bevorzugt sind Cholestyramin (im Handel erhältlich unter der Bezeichnung Qestran® von Bristol-Myers Squibb) und Colestipol (im Handel erhältlich unter der Bezeichnung Colestid® von Pharmacia & Upjohn) (siehe auch *Exp. Opin. Invest. Drugs* (1998), **7**(5), Seiten 715 - 727).

Unter den zuvor genannten Fibrinsäure-Derivaten erfindungsgemäß bevorzugt sind Ciprofibrat (im Handel erhältlich unter der Bezeichnung Modalim® von Sanofi Winthrop), Fenofibrat (im Handel erhältlich unter der Bezeichnung Lipantil® von Fournier), Gemfibrozil (im Handel erhältlich unter der Bezeichnung Lopid® von Parke-Davis), Bezafibrat und Chlofibrat (siehe auch *Exp. Opin. Invest. Drugs* (1998), **7**(5), Seiten 715 - 727).

Unter den zuvor genannten Nikotinsäure-Analogen erfindungsgemäß bevorzugt ist Acipimox (im Handel erhältlich unter der Bezeichnung Olbetam® von Pharmacia & Upjohn) (siehe auch *Exp. Opin. Invest. Drugs* (1998), **7** (5), Seiten 715 - 727).

Unter den zuvor genannten ω3-Fettsäuren erfindungsgemäß bevorzugt ist Maxepa (vertrieben von Seven Seas) (siehe hierzu auch *Exp. Opin. Invest. Drugs* (1998), **7**(5), Seiten 715 - 727).

Die Phosphodiesterase-Hemmern erfindungsgemäß sind cGMP PDE-Inhibitoren. Hierunter wiederum bevorzugt sind insbesondere solche, die oral appliziert werden können und gleichzeitig auch eine gute Wirksamkeit nach oraler Gabe zeigen.

Beispiele für erfindungsgemäß verwendbare und bevorzugte cGMP PDE-Inhibitoren sind insbesondere die in der EP-A-0 463 756, EP-A-0 526 004, WO-A-94/28902 sowie EP-B-0 702 555 beschriebenen Pyrazolopyrimidone. Es handelt sich hierbei insbesondere um Verbindungen der folgenden allgemeinen Formel

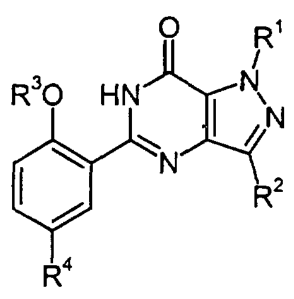

worin

$R^1$ bedeutet: Wasserstoff; $C_1$-$C_3$-Alkyl; $C_1$-$C_3$-Perfluoralkyl; oder $C_3$-$C_5$-Cycloalkyl;

$R^2$ darstellt: Wasserstoff; $C_1$-$C_6$-Alkyl, gegebenenfalls substituiert mit $C_3$-$C_6$-Cycloalkyl; $C_1$-$C_3$-Perfluoralkyl; oder $C_3$-$C_6$-Cycloalkyl;

$R^3$ ist: $C_1$-$C_6$-Alkyl, gegebenenfalls substituiert mit $C_3$-$C_6$-Cycloalkyl; $C_1$-$C_6$-Perfluoralkyl; $C_3$-$C_5$-Cycloalkyl; $C_3$-$C_6$Alkenyl; oder $C_3$-$C_6$-Alkinyl;

$R^4$ bedeutet: $C_1$-$C_4$-Alkyl, gegebenenfalls substituiert mit OH, $NR^5R^6$, CN, $CONR^5R^6$ oder $CO_2R^7$; $C_2$-$C_4$-Alkenyl, gegebenenfalls substituiert mit CN, $CONR^5R^6$ oder $CO_2R^7$; $C_2$-$C_4$-Alkanoyl, gegebenenfalls substituiert mit $NR^5R^6$; (Hydroxy)-$C_2$-$C_4$-alkyl, gegebenenfalls substituiert mit $NR^5R^6$, ($C_2$-$C_3$-Alkoxy)-$C_1$-$C_2$-alkyl, gegebenenfalls substituiert mit OH oder $NR^5R^6$, $CO_2R^7$; Halogen; $NR^5R^6$, $NHSO_2NR^5R^6$; $NHSO_2R^8$; $SO_2NR^9R^{10}$; oder Phenyl, Pyridyl, Pyrimidinyl, Imidazolyl, Oxazolyl, Thiazolyl, Thienyl oder Triazolyl, von denen jedes gegebenenfalls substituiert ist mit Methyl;

$R^5$ und $R^6$ jeweils unabhängig Wasserstoff oder $C_1$-$C_4$-Alkyl darstellen; oder zusammen mit dem Stickstoffatom, an das sie gebunden sind, eine Pyrrolidinyl-, Piperidino-, Morpholino-, 4-N($R^{11}$)-Piperazinyl- oder Imidazolyl-Gruppe bilden, wobei diese Gruppe gegebenenfalls substituiert ist mit Methyl oder OH;

$R^7$ Wasserstoff oder $C_1$-$C_4$-Alkyl ist;

$R^8$ $C_1$-$C_3$-Alkyl, gegebenenfalls substituiert mit $NR^5R^6$, bedeutet;

$R^9$ und $R^{10}$ zusammen mit dem Stickstoffatom, an das sie gebunden sind, eine Pyrrolidinyl-, Piperidino-, Morpholino-, 4-N($R^{12}$)-Piperazinyl-Gruppe bilden, wobei diese Gruppe gegebenenfalls substituiert ist mit $C_1$-$C_4$-Alkyl, $C_1$-$C_3$-Alkoxy, $NP^{13}R^{14}$ oder $CONR^{13}R^{14}$;

$R^{11}$ Wasserstoff, $C_1$-$C_3$-Alkyl, gegebenenfalls substituiert mit Phenyl; (Hydroxy)-$C_2$-$C_3$-alkyl; oder $C_1$-$C_4$-Alkanoyl darstellt

$R^{12}$ Wasserstoff, $C_1$-$C_6$-Alkyl, ($C_1$-$C_3$-Alkoxy)-$C_2$-$C_6$-alkyl; (Hydroxy)-$C_2$-$C_6$alkyl; ($R^{13}$ $R^{14}$N)-$C_2$-$C_6$-Alkyl; ($R^{13}R^{14}$ NOC)-$C_1$-$C_6$-Alkyl; $CONR^{13}R^{14}$; $CSNR^{13}R^{14}$, oder C(NH)$NR^{13}R^{14}$ ist; und

$R^{13}$ und $R^{14}$ jeweils unabhängig Wasserstoff; $C_1$-$C_4$-Alkyl; ($C_1$-$C_3$-Alkoxy)-$C_2$-$C_4$alkyl; oder (Hydroxy)-$C_2$-$C_4$-alkyl bedeuten,

sowie ihre jeweiligen Salze, Hydrate, Alkoholate und Tautomere.

Hierunter erfindungsgemäß ganz besonders bevorzugt sind das 5-[2-Ethoxy-5-(4-methyl-1-piperazinylsulfonyl)-phenyl]-1-methyl-3-n-propyl-6,7-dihydro-1H-pyrazolo-[4,3-d]-pyrimidin-7-on (anderer Name: 5-[2-Ethoxy-5-(4-methyl-1-piperazinylsulfonyl)-phenyl]-1-methyl-3-n-propyl-1,6-dihydro-7H-pyrazolo-{4,3-d]-pyrimidin-7-on, auch als "Sildenafil" bekannt) und dessen Salze, so z.B. insbesondere das Citratsalz, das im Handel unter der Bezeichnung Viagra™ erhältlich ist (siehe insbesondere Beispiel 12 und Verbindung 3 des Anspruchs 3 der EP-A-0 463 756 sowie Anspruch 6 der EP-B-0 702 555).

Als cGMP PDE-Inhibitor erfindungsgemäß ebenfalls sind die in der WO-A-99/24433 beschriebenen Verbindungen. Es handelt sich hierbei um 2-phenylsubstituierte Imidazotriazinone der allgemeinen Formel

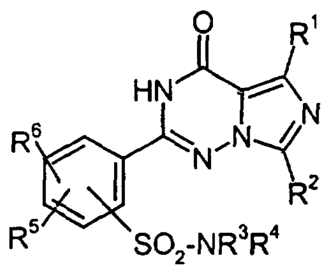

(I)

in welcher

$R^1$ für Wasserstoff oder geradkettiges oder verzweigtes Alkyl mit bis zu 4 Kohlenstoffatomen steht;

$R^2$ für geradkettiges Alkyl mit bis zu 4 Kohlenstoffatomen steht;

$R^3$ und $R^4$ gleich oder verschieden sind und für Wasserstoff oder für geradkettiges oder verzweigtes Alkenyl oder Alkoxy mit jeweils bis zu 8 Kohlenstoffatomen stehen, oder

für eine geradkettige oder verzweigte Alkylkette mit bis zu 10 Kohlenstoffatomen stehen, die gegebenenfalls durch ein Sauerstoffatom unterbrochen ist, und die gegebenenfalls ein- bis mehrfach, gleich oder verschieden durch Trifluormethyl, Trifluormethoxy, Hydroxy, Halogen, Carboxyl, Benzyloxycarbonyl, geradkettiges oder verzweigtes Alkoxycarbonyl mit bis zu 6 Kohlenstoffatomen und/oder durch Reste der Formeln $-SO_3H$, $-(A)_a-NR^7R^8$, $-O-CO-NR^{7'}R^{8'}$, $-S(O)_b-R^9$, $-P(O)(OR^{10})(OR^{11})$,

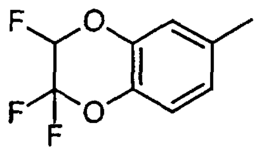 , 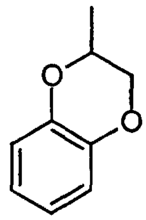 ,

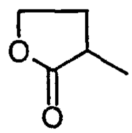 und/oder 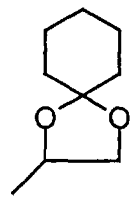

substituiert ist,
worin

a und b gleich oder verschieden sind und eine Zahl 0 oder 1 bedeuten,

A einen Rest CO oder $SO_2$ bedeutet,

$R^7$, $R^{7'}$, $R^8$ und $R^{8'}$ gleich oder verschieden sind und Wasserstoff bedeuten, oder Cycloalkyl mit 3 bis 8 Kohlenstoffatomen, Aryl mit 6 bis 10 Kohlenstoffatomen, einen 5- bis 6-gliedrigen ungesättigten, partiell ungesättigten oder gesättigten, gegebenenfalls benzokondensierten Heterocyclus, mit bis zu 3 Heteroatomen aus der Reihe S, N und/oder O bedeuten, wobei die oben aufgeführten Ringsysteme gegebenenfalls ein- bis mehrfach, gleich oder verschieden durch Hydroxy, Nitro, Trifluormethyl, Trifluormethoxy, Carboxyl, Halogen, geradkettiges oder verzweigtes Alkoxy oder Alkoxycarbonyl mit jeweils bis zu 6 Kohlenstoffatomen oder durch eine Gruppe der Formel $-(SO_2)_c-NR^{12}R^{13}$ substituiert sind,
worin

c eine Zahl 0 oder 1 bedeutet,

$R^{12}$ und $R^{13}$ gleich oder verschieden sind und Wasserstoff oder geradkettiges oder verzweigtes Alkyl mit bis zu 5 Kohlenstoffatomen bedeuten,

oder

$R^7$, $R^{7'}$, $R^8$ und $R^{8'}$ geradkettiges oder verzweigtes Alkoxy mit bis zu 6 Kohlenstoffatomen bedeuten, oder geradkettiges oder verzweigtes Alkyl mit bis zu 8 Kohlenstoffatomen bedeuten, das gegebenenfalls ein- oder mehrfach, gleich oder verschieden durch Hydroxy, Halogen, Aryl mit 6 bis 10 Kohlenstoffatomen, geradkettiges oder verzweigtes Alkoxy oder Alkoxycarbonyl mit jeweils bis zu 6 Kohlenstoffatomen oder durch eine Gruppe der Formel $-(CO)_d-NR^{14}R^{15}$ substituiert ist,
worin

$R^{14}$ und $R^{15}$ gleich oder verschieden sind und Wasserstoff oder geradkettiges oder verzweigtes Alkyl mit bis zu 4 Kohlenstoffatomen bedeuten,

und

d eine Zahl 0 oder 1 bedeutet,

oder

$R^7$ und $R^8$ und/oder $R^{7'}$ und $R^{8'}$ gemeinsam mit dem Stickstoffatom einen 5-bis 7-gliedrigen, gesättigten Heterocyclus bilden, der gegebenenfalls noch ein weiteres Heteroatom aus der Reihe S oder O oder einen Rest der Formel $-NR^{16}$ enthalten kann,
worin

$R^{16}$ Wasserstoff, Aryl mit 6 bis 10 Kohlenstoffatomen, Benzyl, einen 5- bis 7-gliedrigen aromatischen oder gesättigten Heterocyclus mit bis zu 3 Heteroatomen aus der Reihe S, N und/oder O bedeutet, der gegebenenfalls durch Methyl substituiert ist, oder
geradkettiges oder verzweigtes Alkyl mit bis zu 6 Kohlenstoffatomen bedeutet, das gegebenenfalls durch Hydroxy substituiert ist,

$R^9$ Aryl mit 6 bis 10 Kohlenstoffatomen bedeutet, oder
geradkettiges oder verzweigtes Alkyl mit bis zu 4 Kohlenstoffatomen bedeutet,

$R^{10}$ und $R^{11}$ gleich oder verschieden sind und Wasserstoff oder geradkettiges oder verzweigtes Alkyl mit bis zu 4 Kohlenstoffatomen bedeuten,

und/oder die oben unter $R^3$/$R^4$ aufgeführte Alkylkette gegebenenfalls durch Cycloalkyl mit 3 bis 8 Kohlenstoffatomen, Aryl mit 6 bis 10 Kohlenstoffatomen oder durch einen 5- bis 7-gliedrigen, partiell ungesättigten, gesättigten oder ungesättigten, gegebenenfalls benzokondensierten Heterocyclus, der bis zu 4 Heteroatome aus der Reihe S, N; O oder einen Rest der Formel $-NR^{17}$ enthalten kann, substituiert ist,
worin

$R^{17}$ Wasserstoff, Hydroxy, Formyl, Trifluormethyl, geradkettiges oder verzweigtes Acyl oder Alkoxy mit jeweils bis zu 4 Kohlenstoffatomen bedeutet,
oder geradkettiges oder verzweigtes Alkyl mit bis zu 6 Kohlenstoffatomen bedeutet, das gegebenenfalls ein- bis mehrfach, gleich oder verschieden durch Hydroxy, oder geradkettiges oder verzweigtes Alkoxy mit bis zu 6 Kohlenstoffatomen substituiert ist,

und wobei Aryl und der Heterocyclus gegebenenfalls ein- bis mehrfach, gleich oder verschieden durch Nitro, Halogen, $-SO_3H$, geradkettiges oder verzweigtes Alkyl oder Alkoxy mit jeweils bis zu 6 Kohlenstoffatomen, Hydroxy, Trifluormethyl, Trifluormethoxy und/oder durch einen Rest der Formel $-SO_2NR^{18}R^{19}$ substituiert sind,
worin

$R^{18}$ und $R^{19}$ gleich oder verschieden sind und Wasserstoff oder geradkettiges oder verzweigtes Alkyl mit bis zu 6 Kohlenstoffatomen bedeuten,

und/oder

$R^3$ oder $R^4$ für eine Gruppe der Formel $-NR^{20}R^{21}$ steht,
worin

$R^{20}$ und $R^{21}$ die oben angegebene Bedeutung von $R^{18}$ und $R^{19}$ haben und mit dieser gleich oder verschieden sind,

und/oder

$R^3$ oder $R^4$ für Adamantyl stehen, oder
für Reste der Formeln

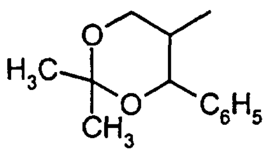 , 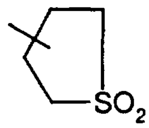 , 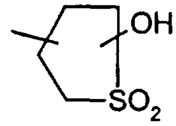

oder

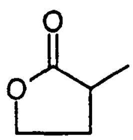

stehen,
oder für Cycloalkyl mit 3 bis 8 Kohlenstoffatomen, Aryl mit 6 bis 10 Kohlenstoffatomen oder für einen 5- bis 7-gliedrigen partiell ungesättigten, gesättigten oder ungesättigten, gegebenenfalls benzokondensierten Heterocyclus stehen, der bis zu 4 Heteroatome aus der Reihe S, N; O oder einen Rest der Formel $-NR^{22}$ enthalten kann,
worin

$R^{22}$ die oben angegebene Bedeutung von $R^{16}$ hat und mit dieser gleich oder verschieden ist, oder Carboxyl, Formyl oder geradkettiges oder verzweigtes Acyl mit bis zu 5 Kohlenstoffatomen bedeutet,

und wobei Cycloalkyl, Aryl und/oder der Heterocyclus gegebenenfalls einbis mehrfach, gleich oder verschieden durch Halogen, Triazolyl, Trifluormethyl, Trifluormethoxy, Carboxyl, geradkettiges oder verzweigtes Acyl oder Alkoxycarbonyl mit jeweils bis zu 6 Kohlenstoffatomen, Nitro und/oder durch Gruppen der Formeln $-SO_3H$, $-OR^{23}$, $(SO_2)_cNR^{24}R^{25}$, $-P(O)(OR^{26})(OR^{27})$ substituiert sind,
worin

e eine Zahl 0 oder 1 bedeutet,

$R^{23}$ einen Rest der Formel

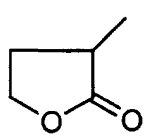

bedeutet, oder
Cycloalkyl mit 3 bis 7 Kohlenstoffatomen bedeutet, oder
Wasserstoff oder geradkettiges oder verzweigtes Alkyl mit bis zu 4 Kohlenstoffatomen bedeutet, das gegebenenfalls durch Cycloalkyl mit 3 bis 7 Kohlenstoffatomen, Benzyloxy, Tetrahydropyranyl, Tetrahydrofuranyl, geradkettiges oder verzweigtes Alkoxy oder Alkoxycarbonyl mit jeweils bis zu 6 Kohlenstoffatomen, Carboxyl, Benzyloxycarbonyl oder Phenyl substituiert ist, das seinerseits ein- bis mehrfach, gleich oder verschieden durch geradkettiges oder verzweigtes Alkoxy mit bis zu 4 Kohlenstoffatomen, Hydroxy oder Halogen substituiert sein kann,
und/oder Alkyl gegebenenfalls durch Reste der Formeln $-CO-NR^{28}R^{29}$ oder $-CO-R^{30}$ substituiert ist,
worin

$R^{28}$ und $R^{29}$ gleich oder verschieden sind und Wasserstoff oder geradkettiges oder verzweigtes Alkyl mit bis zu 8 Kohlenstoffatomen bedeuten, oder

$R^{28}$ und $R^{29}$ gemeinsam mit dem Stickstoffatom einen 5- bis 7-gliedrigen gesättigten Heterocyclus bilden, der gegebenenfalls ein weiteres Heteroatom aus der Reihe S oder O enthalten kann, und

$R^{30}$ Phenyl oder Adamantyl bedeutet,

$R^{24}$ und $R^{25}$ die oben angegebene Bedeutung von $R^{18}$ und $R^{19}$ haben und mit dieser gleich oder verschieden sind,

$R^{26}$ und $R^{27}$ die oben angegebene Bedeutung von $R^{10}$ und $R^{11}$ haben und mit dieser gleich oder verschieden sind

und/oder Cycloalkyl, Aryl und/oder der Heterocyclus gegebenenfalls durch geradkettiges oder verzweigtes Alkyl mit bis zu 6 Kohlenstoffatomen substituiert sind, das gegebenenfalls durch Hydroxy, Carboxyl, durch einen 5-bis 7-gliedrigen Heterocyclus mit bis zu 3 Heteroatomen aus der Reihe S, N und/oder O oder durch Gruppen der Formel $-SO_2-R^{31}$, $P(O)(OR^{32})(OR^{33})$ oder $-NR^{34}R^{35}$ substituiert ist,
worin

$R^{31}$ Wasserstoff bedeutet oder die oben angegebene Bedeutung von $R^9$ hat und mit dieser gleich oder verschieden ist,

$R^{32}$ und $R^{33}$ die oben angegebene Bedeutung von $R^{10}$ und $R^{11}$ haben und mit dieser gleich oder verschieden sind,

$R^{34}$ und $R^{35}$ gleich oder verschieden sind und Wasserstoff oder geradkettiges oder verzweigtes Alkyl mit bis zu 6 Kohlenstoffatomen bedeuten, das gegebenenfalls durch Hydroxy oder geradkettiges oder verzweigtes Alkoxy mit bis zu 4 Kohlenstoffatomen substituiert ist, oder

$R^{34}$ und $R^{35}$ gemeinsam mit dem Stickstoffatom einen 5- bis 6-gliedrigen gesättigten Heterocyclus bilden, der ein weiteres Heteroatom aus der Reihe S oder O oder einen Rest der Formel $-NR^{36}$ enthalten kann,
worin

$R^{36}$ Wasserstoff, Hydroxy, geradkettiges oder verzweigtes Alkoxycarbonyl mit bis zu 7 Kohlenstoffatomen oder geradkettiges oder verzweigtes Alkyl mit bis zu 5 Kohlenstoffatomen bedeutet, das gegebenenfalls durch Hydroxy substituiert ist,

oder

$R^3$ und $R^4$ gemeinsam mit dem Stickstoffatom einen 5- bis 7-gliedrigen, ungesättigten oder gesättigten oder partiell ungesättigten, gegebenenfalls benzokondensierten Heterocyclus bilden, der gegebenenfalls bis zu 3 Heteroatome aus der Reihe S, N, O oder einen Rest der Formel $-NR^{37}$ enthalten kann,
worin

$R^{37}$ Wasserstoff, Hydroxy, Formyl, Trifluormethyl, geradkettiges oder verzweigtes Acyl, Alkoxy oder Alkoxycarbonyl mit jeweils bis zu 4 Kohlenstoffatomen bedeutet,
oder geradkettiges oder verzweigtes Alkyl mit bis zu 6 Kohlenstoffatomen bedeutet, das gegebenenfalls ein- bis mehrfach, gleich oder verschieden durch Hydroxy, Trifluormethyl, Carboxyl, geradkettiges oder verzweigtes Alkoxy oder Alkoxycarbonyl mit jeweils bis zu 6 Kohlenstoffatomen oder durch Gruppen der Formel $-(D)_fNW^{38}R^{39}$, $-CO-(CH_2)_g-O-CO-R^{40}$, $-CO-(CH_2)_h-OR^{41}$ oder $-P(O)(OR^{42})(OR^{43})$ substituiert ist,
worin

g und h gleich oder verschieden sind und eine Zahl 1, 2, 3 oder 4 bedeuten,

und

f eine Zahl 0 oder 1 bedeutet,

D eine Gruppe der Formel -CO oder $-SO_2$ bedeutet,

$R^{38}$ und $R^{39}$ gleich oder verschieden sind und die oben angegebene Bedeutung von $R^7$ und $R^8$ haben,

$R^{40}$ geradkettiges oder verzweigtes Alkyl mit bis zu 6 Kohlenstoffatomen bedeutet,

$R^{41}$ geradkettiges oder verzweigtes Alkyl mit bis zu 6 Kohlenstoffatomen bedeutet,

$R^{42}$ und $R^{43}$ gleich oder verschieden sind und Wasserstoff oder geradkettiges oder verzweigtes Alkyl mit bis zu 4 Kohlenstoffatomen bedeuten,

oder

$R^{37}$ einen Rest der Formel $-(CO)_i-E$ bedeutet,
worin

i eine Zahl 0 oder 1 bedeutet,

E Cycloalkyl mit 3 bis 7 Kohlenstoffatomen oder Benzyl bedeutet,
Aryl mit 6 bis 10 Kohlenstoffatomen oder einen 5- bis 6-gliedrigen aromatischen Heterocyclus mit bis zu 4 Heteroatomen aus der Reihe S, N und/oder O bedeutet, wobei die oben aufgeführten Ringsysteme gegebenenfalls ein- bis mehrfach, gleich oder verschieden durch Nitro, Halogen, $-SO_3H$, geradkettiges oder verzweigtes Alkoxy mit bis zu 6 Kohlenstoffatomen, Hydroxy, Trifluormethyl, Trifluormethoxy oder durch einen Rest der Formel $-SO_2-NR^{44}R^{45}$, substituiert sind,
worin

$R^{44}$ und $R^{45}$ die oben angegebene Bedeutung von $R^{18}$ und $R^{19}$ haben und mit dieser gleich oder verschieden sind,

oder

E Reste der Formeln

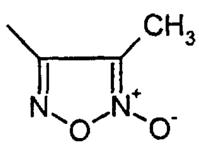

,

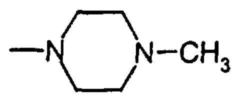

oder

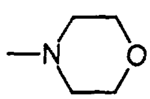

bedeutet,

und der unter $R^3$ und $R^4$ aufgeführte, gemeinsam mit dem Stickstoffatom gebildete Heterocyclus, gegebenenfalls ein- bis mehrfach, gleich oder verschieden, gegebenenfalls auch geminal, durch Hydroxy, Formyl, Carboxyl, geradkettiges oder verzweigtes Acyl oder Alkoxycarbonyl mit bis jeweils zu 6 Kohlenstoffatomen, Nitro und Gruppen der Formeln $-P(O)(OR^{46})(OR^{47})$,

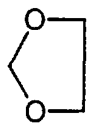,

$$=NR^{48} \text{ oder } -(CO)_jNR^{49}R^{50}$$

substituiert ist,
worin

$R^{46}$ und $R^{47}$ die oben angegebene Bedeutung von $R^{10}$ und $R^{11}$ haben und mit dieser gleich oder verschieden sind,

$R^{48}$ Hydroxy oder geradkettiges oder verzweigtes Alkoxy mit bis zu 4 Kohlenstoffatomen bedeutet,

j eine Zahl 0 oder 1 bedeutet,
und

$R^{49}$ und $R^{50}$ gleich oder verschieden sind und die oben angegebene Bedeutung von $R^{14}$ und $R^{15}$ haben,

und/oder der unter $R^3$ und $R^4$ aufgeführte, gemeinsam mit dem Stickstoffatom gebildete Heterocyclus, gegebenenfalls durch geradkettiges oder verzweigtes Alkyl mit bis zu 6 Kohlenstoffatomen substituiert ist, das gegebenenfalls einbis mehrfach, gleich oder verschieden durch Hydroxy, Halogen, Carboxyl, Cycloalkyl oder Cycloalkyloxy mit jeweils 3 bis 8 Kohlenstoffatomen, geradkettiges oder verzweigtes Alkoxy oder Alkoxycarbonyl mit jeweils bis zu 6 Kohlenstoffatomen oder durch einen Rest der Formel $-SO_3H$, $-NR^{51}R^{52}$ oder $P(O)OR^{53}OR^{54}$ substituiert ist,
worin

$R^{51}$ und $R^{52}$ gleich oder verschieden sind und Wasserstoff, Phenyl, Carboxyl, Benzyl oder geradkettiges oder verzweigtes Alkyl oder Alkoxy mit jeweils bis zu 6 Kohlenstoffatomen bedeuten,

$R^{53}$ und $R^{54}$ gleich oder verschieden sind und die oben angegebene Bedeutung von $R^{10}$ und $R^{11}$ haben,

und/oder das Alkyl gegebenenfalls durch Aryl mit 6 bis 10 Kohlenstoffatomen substituiert ist, das seinerseits ein- bis mehrfach, gleich oder verschieden durch Halogen, Hydroxy, geradkettiges oder verzweigtes Alkoxy mit bis zu 6 Kohlenstoffatomen, oder durch eine Gruppe der Formel $-NR^{51'}R^{52'}$ substituiert sein kann,
worin

$R^{51'}$ und $R^{52'}$ die oben angegebene Bedeutung von $R^{51}$ und $R^{52}$ haben und mit dieser gleich oder verschieden sind,

und/oder der unter $R^3$ und $R^4$ aufgeführte, gemeinsam mit dem Stickstoffatom gebildete Heterocyclus, gegebenenfalls durch Aryl mit 6 bis 10 Kohlenstoffatomen oder durch einen 5- bis 7-gliedrigen, gesättigen, partiell ungesättigten oder ungesättigten Heterocyclus mit bis zu 3 Heteroatomen aus der Reihe S, N und/oder O , gegebenenfalls auch über eine N-Funktion verknüpft, substituiert ist, wobei die Ringsysteme ihrerseits durch Hydroxy oder durch geradkettiges oder verzweigtes Alkyl oder Alkoxy mit jeweils bis zu 6 Kohlenstoffatomen substituiert sein können,
oder

$R^3$ und $R^4$ gemeinsam mit dem Stickstoffatom Reste der Formeln

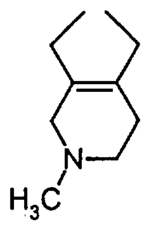 , 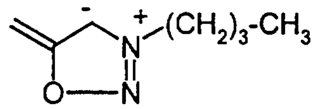 , 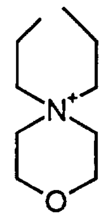

oder

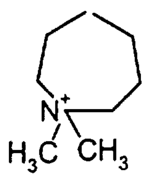

bilden,

$R^5$ und $R^6$ gleich oder verschieden sind und für Wasserstoff, geradkettiges oder verzweigtes Alkyl mit bis zu 6 Kohlenstoffatomen, Hydroxy oder für geradkettiges oder verzweigtes Alkoxy mit bis zu 6 Kohlenstoffatomen stehen,

sowie ihre jeweiligen Salze, Hydrate, Alkoholate und Tautomere.

Unter den in der WO-A-99/24433 genannten Verbindungen erfindungsgemäß ganz besonders bevorzugt sind das 2-[2-Ethoxy-5-(4-ethyl-piperazin-1-sulfonyl)-phenyl]-5-methyl-7-propyl-3H-imidazo[5,1-f][1,2,4]triazin-4-on (Bsp. 19 der WO-A-99/24433) und seine Salze, so z.B. das Hydrochlorid (Bsp. 20 der WO-A-99/24433), insbesondere in der Form des Trihydrats (Bsp. 336 der WO-A-99/24433).

Weitere erfindungsgemäß bevorzugte PDE V-Inhibitoren sind (a) Zaprinast *(Am. J. Physiol.,* Vol. 264, Februar 1993, Seiten H419-H422; *The Journal of Urology,* Vol. 147, Seiten 1650-1655 (Juni 1992); *The New England Journal of Medicine,* Vol. 326(2), Seiten 90-94 (9. Januar 1992)); (b) Propentofyllin (JP-A-03/044324); und (c) Pentoxifyllin *(Postgraduate Medicine,* Vol. 93, No. 3, Impotence, 15. Februar 1993, Seiten 65-72; *J.A.G.S.,* **41**, Seiten 363-366, 1993).

Weitere erfindungsgemäß bevorzugte PDE V-Inbibitoren sind in den folgenden Druckschriften offenbart:

- 5-Substituierte Pyrazolo-[4,3-d]-pyrimidin-7-one gemäß EP-A-0 201 188;
- Griseolsäurederivate gemäß EP-A-0 214 708 und EP-A-0 319 050;
- 2-Phenylpurinonderivate gemäß EP-A-0 293 063;
- Phenylpyridonderivate gemäß EP-A-0 347 027;
- Anellierte Pyrimidinderivate gemäß EP-A-0 347 146;
- Kondensierte Pyrimidinderivate gemäß EP-A-0 349 239;
- Pyrimidopyrimidinderivate gemäß EP-A-0 351 058;
- Purinverbindungen gemäß EP-A-0 352 960;
- Chinazolinderivate gemäß EP-A-0 371 731;
- Phenylpyrimidonderivate gemäß EP-A-0 395 328;
- Imidazochinoxalinonderivate oder ihre Azaanalogen gemäß EP-A-0 400 583;
- Phenylpyrimidonderivate gemäß EP-A-0 400 799;
- Phenylpyridonderivate gemäß EP-A-0 428 268;
- Pyrimidopyrimidinderivate gemäß EP-A-0 442 204;

- 4-Aminochinazolinderivate gemäß EP-A-0 579 496;
- 4,5-Dihydro-4-oxo-pyrrolo-[1,2-a]-chinoxalinderivate und ihre Azaanalogen gemäß EP-A-0 584 487;
- Polycyclische Guaninderivate gemäß WO-A-91/19717;
- Stickstoffhaltige Heterocyclen gemäß WO-A-93/07124;
- Polycyclische 2-Benzyl-Guaninderivate gemäß WO-A-94/19351;
- Chinazolinderivate gemäß US-A-4 060 615;
- 6-Heterocyclyl-pyrazolo-[3,4-d]-pyrimidin-4-one gemäß US-A-5 294 612;
- Benzimidazole gemäß JP-A 5-222000;
- Cycloheptimidazol gemäß *European Journal of Pharmacology* 1994, **251,** Seite 1;
- N-enthaltende Heterocyclen gemäß WO-A-94/22855.

Weitere erfindungsgemäß verwendbare und bevorzugte PDE-Inhibitoren sind:

- Tetracyclische Derivate gemäß WO-A-95/19978;
- Pyrazolopyrimidin-Derivate gemäß EP-A-0 636 626;
- 4-Aminopyrimidin-Derivate gemäß EP-A-0 640 599;
- Imidazochinazolin-Derivate gemäß EP-A-0 668 280;
- Chinazolinverbindungen gemäß EP-A-0 669 324;
- 4-Aminochinazolin-Derivate gemäß US-A-5 436 233.

Weitere erfindungsgemäß verwendbare und gleichermaßen bevorzugte PDE-Inhibitoren sind die Verbindungen gemäß EP-A-0 579 496; WO-A-93/07124; US-A-5 294 612 und WO-A-94/22855.

Beispiele für erfindungsgemäß besonders bevorzugte PDE-Hemmer aus den zuvor genannten Druckschriften sind die folgenden Verbindungen:

- 1,3-Dimethyl-5-benzylpyrazolo-[4,3-d]-pyrimidin-7-on (Herstellung gemäß EP-A-0 201 188, Beispiel 1);
- 2-(2-Propoxyphenyl)-6-purinon (Herstellung gemäß EP-A-0 293 063, Beispiel 1);
- 6-(2-Propoxyphenyl)-1,2-dihydro-2-oxopyridin-3-carboxamid (Herstellung gemäß EP-A-0 347 027, Beispiel 2);
- 2-(2-Propoxyphenyl)-pyrido-[2,3-d]-pyrimid-4(3H)-on (Herstellung gemäß EP-A-0 347 146, Beispiel 1);
- 7-Methylthio-4-oxo-2-(2-propoxyphenyl)-3,4-dihydropyrimido-[4,5-d]-pyrimidin (Herstellung gemäß EP-A-0 351 058, Beispiel 1);
- 6-Hydroxy-2-(2-propoxyphenyl)-pyrimidin-4-carboxamid (Herstellung gemäß EP-A-0 395 328, Beispiel 15);
- 1-Ethyl-3-methylimidazo-[1,5-a]-chinoxalin-4(5H)-on (Herstellung gemäß EP-A-0 400 583);
- 4-Phenylmethylamino-6-chlor-2-(1-imidazolyl)-chinoxalin (Herstellung gemäß EP-A-0 579 496, Beispiel 5(c));
- 5-Ethyl-8-[3-(N-cyclohexyl-N-methylcarbamyl)-propyloxy]-4,5-dihydro-4-oxo-pyrido-[3,2-e]-pyrrolo-[1,2-a]-pyrazin (Herstellung gemäß EP-A-0 584 487, Beispiel 1);
- 5'-Methyl-3'-(phenylmethyl)-spiro-[cyclopentan-1,7'(8'H)-(3'H)-imidazo[2,1-b]-purin]-4'(5'H)-on (Herstellung gemäß WO-A-91/19717, Beispiel 9A3);
- 1-[6-Chlor-4-(3,4-methylendioxybenzyl)-aminochinazolin-2-yl)-piperidin-4-carbonsäure (Herstellung gemäß WO-A-93/97124);
- (6aR,9aS)-2-(4-Trifluormethylphenyl)-methyl-5-methyl-3,4,5,6a,7,8,9,9a-octahydrocyclopent-[4,5]-imidazo-[2,1-b]-purin-4-on (Herstellung gemäß WO-A-94/19351, Beispiel 14);
- 1-tert.-Butyl-3-phenylmethyl-6-(4-pyridyl)-pyrazolo-[3,4-d]-pyrimid-4-on (Herstellung gemäß US-A-5 294 612, Beispiel 90);
- 1-Cyclopentyl-3-methyl-6-(4-pyridyl)-4,S-dihydro-1H-pyrazolo-[3,4-d]-pyrimid-4-on (Herstellung gemäß US-A-5 294 612, Beispiel 83);
- 2-Butyl-1-(2-chlorbenzyl)-6-ethoxycarbonylbenzimidazol (Herstellung gemäß JP-A-5-222000);
- 2-(4-Carboxypiperidino)-4-(3,4-methylendioxybenzyl)-amino-6-nitrochinazolin (Herstellung gemäß WO-A-94/22855, Beispiel II);
- 2-Phenyl-8-ethoxycycloheptimidazol (KT2-734);
- 1-[6-Chlor-4-(3,4-methyldioxybenzyl)-aminochinazolin-2-yl]-piperidin-4-carbonsäure (Herstellung gemäß WO-A-93/07124);
- (6aR,9aS)-2-(4-Trifluormethylphenyl)-methyl-5-methyl-3,4,S,6a,7,8,9,9a-octahydrocyclopent-[4,S]-imidazo-[2,1-b]-purin-4-on (Herstellung gemäß WO-A-94/19351, Beispiel 14);
- 1-tert.-Butyl-3-phenylmethyl-6-(4-pyridyl)-pyrazolo-[3,4-d]-pyrimid-4-on (Herstellung gemäß US-A-5 294 612, Beispiel 90);
- 1-Cyclopentyl-3-methyl-6-(4-pyridyl-4,5-dihydro-1H-pyrazolo-[3,4-d]-pyrimid-4-on (Herstellung gemäß US-A-5 294 612, Beispiel 83);

- 2-(4-Carboxypiperidino)-4-(3,4-methylendioxybenzyl)-amino-6-nitro-chinazolin (Herstellung gemäß WO-A-94/22855, Beispiel II).

Weitere erfindungsgemäß verwendbare cGMP PDE-Inhibitoren sind die folgenden Verbindungen:

- Pyrazolopyrimidin-Derivate gemäß EP-A-0 636 626;
- 4-Aminopyrimidin-Derivate gemäß EP-A-0 640 599;
- Imidazochinazolin-Derivate gemäß WO-A-95/06648;
- Anthranilsäure-Derivate gemäß WO-A-95/18097;
- 4-Aminochinazolin-Derivate gemäß US-A-5 436 233;
- Tetracyclische Derivate gemäß WO-A-95/19978;
- Imidazochinazolin-Derivate gemäß EP-A-0 668 280;
- Chinazolinverbindungen gemäß EP-A-0 669 324.

Weitere erfindungsgemäß bevorzugte PDE-Inhibitoren, insbesondere cGMP PDE-Inhibitoren, sind in den folgenden Druckschriften offenbart, deren Inhalt hiermit durch Bezugnahme eingeschlossen ist:

- Chinolonderivate gemäß WO-A-98/53819;
- Pyrazolo[4,3-d]-pyrimidin-Derivate gemäß EP-A-911333;
- Pyrazolopyrimidone gemäß WO-A-98/49166;
- Verbindungen gemäß WO-A-96/16644;
- Bicyclische Heterocyclen gemäß WO-A-96/16657;
- Pyrazolopyrimidone gemäß WO-A-93/06104;
- Pyrazolopyrimidone gemäß WO-A-93/07149;
- Chinazoline gemäß WO-A-93/12093;
- Purinone gemäß WO-A-94/00453;
- Pyridopyrimidinone gemäß WO-A-94/05661;
- Verbindungen gemäß US-A-5 272 147 und US-A-5 250 534; und
- Chinazolinone gemäß WO-A-93/12095.

Weitere erfindungsgemäß bevorzugte PDEV-Inhibitoren sind ausgewählt aus der Gruppe von:

- 4-(3-Chlor-4-methoxybenzyl)amino-1-(4-hydroxypiperidino)-6-phthalazincarbonitril und dessen Salzen, Tautomeren, Alkoholaten und Hydraten, insbesondere 4-(3-Chlor-4-methoxybenzyl)-amino-1-(4-hydroxypiperidino)-6-phthalazincarbonitrilhydrochlorid;
- 1-[4-[(1,3-Benzodioxol-5-ylmethyl)amino]-6-chlor-2-chinazolyl]-4-piperidincarbonsäure und dessen Salzen, Tautomeren, Alkoholaten und Hydraten, insbesondere dem Natriumsalz;
- 4-Brom-6-[3-(4-chlorphenyl)propoxy]-5-[(3-piperidinylmethyl)amino]-3(2H)-pyridazinon und dessen Salzen, Tautomeren, Alkoholaten und Hydraten, insbesondere 4-Brom-6-[3-(4-chlorphenyl)propoxy]-5-[(3-piperidinylmethyl)-amino]-3(2H)-pyridazinonhydrochlorid.

Weitere erfindungsgemäß bevorzugte PDEV-Inhibitoren sind in den folgenden Druckschriften offenbart:

- Carbazolderivate und deren Salze gemäß WO-A-99/21831 (Fujisawa);
- Dihydropyrazolopyrimidinonderivate gemäß WO-A-98/40384 (BAYER) und DE-A-197 09 877 (BAYER);
- Purin- und Pyrazolopyrimidinderivate gemäß DE-A-197 09 126 (BAYER);
- Isochinolinonderivate gemäß WO-A-98/38168 (Tanabe);
- substituierte Azaindolverbindungen gemäß JP-A-10120681 (Fujisawa);
- Indolizinverbindungen gemäß JP-A-10120680 (Fujisawa);
- (Amino-thieno-pyrimidinyl)-heterocyclische Säurederivate gemäß DE-A-196 44 228 (Merck) und WO-A-98/17668 (Merck);
- 2-Amino-thiophen-3-carboxamidderivate gemäß DE-A-196 42 451 (Merck) und WO-A-98/16521 (Merck);
- Pyridocarbazolderivate gemäß WO-A-97/45427 (Mochida);
- Purinonderivate gemäß EP-A-771799 (BAYER);
- N-Benzyl-3-indenylacetamidderivate gemäß WO-A-99/31065 (Baverstock/Cell Pathways/Univ. Arizona State);
- Pyridocarbazolverbindungen gemäß WO-A-99126946 (Mochida);
- Thienopyrimidinderivate gemäß DE-A-197 52 952 (Merck) und WO-A-99/28325 (Merck);
- Indolderivate gemäß WO-A-98/15530 (Fujisawa);

- Pyrazolopyridazinone gemäß WO-A-98/14448 (Kyorin);
- Phenylalkylthienopyrimidinderivate gemäß DE-A-196 32 423 (Merck) und WO-A-98/06722 (Merck);
- Verbindungen gemäß WO-A-96/21435 (Euroceltique);
- Dihydropyrazolopyrrole gemäß WO-A-95/19362 (Pfizer);
- Verbindungen gemäß WO-A-95/00516 (Euroceltique).

Außer den beiden zuvor genannten Wirkstoffkomponenten A und B kann das erfindungsgemäße Kombinationspräparat noch weitere beliebige Wirkstoffe enthalten, sofern, diese nicht dem Indikationsgebiet zuwiderlaufen und nicht die Wirkung des PDEV-Hemmers und des Lipidsenkers beeinträchtigen.

Diese weiteren, gegebenenfalls vorhandenen Wirkstoffe können - wie schon die Wirkstoffkomponenten A und B - entweder als echte Mischung zusammen mit A und/oder B vorliegen oder aber auch räumlich getrennt hiervon vorliegen. Ihre Verabreichung kann parallel oder gleichzeitig oder zeitlich abgestuft zu der/den Wirkstoffkomponente (n) A und/oder B erfolgen.

Zu den weiteren, gegebenenfalls vorhandenen Wirkstoffen des erfindungsgemäßen Kombinationspräparates zählen beispielsweise:

- weitere, die Erektionsfähigkeit verbessernde Wirkstoffe, die nicht zu der Klasse der PDE-Hemmer gehören, so z. B.: $\alpha$-adrenergische Antagonisten wie z.B. Yohimbin oder Vasomax® von der Firma Zonagen; oder auch solche Substanzen, wie sie in der WO-A-98/52569 genannt sind, deren Inhalt hiermit durch Bezugnahme eingeschlossen ist; oder Prostaglandine-E1; oder Seretonin-Antagonisten;
- Wirkstoffe aus dem kardiovaskulären Indikationsbereich;
- Wirkstoffe aus dem ZNS- und cerebralen Indikationsbereich;
- Vitamine;
- Mineralstoffe;
- Spurenelemente.

Für die Applikation der beiden Wirkstoffkomponenten A und B (und der gegebenenfalls vorhandenen weiteren Wirkstoffe) kommen jeweils alle üblichen Applikationsformen in Betracht. Vorzugsweise erfolgt die Applikation oral, perlingual, sublingual, nasal, transdermal, buccal, intravenös, rektal, inhalativ oder parenteral. Vorzugsweise erfolgt die Applikation oral, sublingual oder nasal. Ganz besonders bevorzugt ist die orale Applikation.

Des weiteren ist es möglich, die beiden Wirkstoffkomponenten A und B bei räumlicher getrennter bzw. zeitlich versetzter Verabreichung in unterschiedlicher Darreichungsform zu applizieren.

Die beiden Wirkstoffkomponenten A und B können - zusammen oder räumlich getrennt - jeweils in an sich bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei sollten die therapeutisch wirksamen Komponenten A und B jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der beiden Wirkstoffkomponenten A und B mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Für die Anwendung beim Menschen werden bei oraler Administration Dosierungen von 0,001 bis 50 mg/kg, vorzugsweise von 0,001 mg/kg bis 20 mg/kg, insbesondere 0,001 bis 10 mg/kg Körpergewicht, besonders bevorzugt 0,001 mg/kg bis 5 mg/kg, der jeweiligen Wirkstoffkomponente A oder B zur Erzielung wirksamer und sinnvoller Ergebnisse verabreicht.

Trotzdem kann es gegebenenfalls erforderlich sein, von den hier genannten Mengen abzuweichen, und zwar in Abhängigkeit vom Körpergewicht bzw. von der Art des Applikationsweges, vom individuellen Verhalten gegenüber dem Kombinationspräparat, von der Art der Formulierung und von dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss.

Im Falle der Applikation größerer Mengen kann es empfehlenswert sein, diese in mehreren Einzelgaben über den Tag zu verteilen.

Die vorliegende Erfindung wird durch das folgende Ausführungsbeispiel veranschaulicht.

## Beispiel

In einer placebokontrollierten Studie wurden zwölf adulte, männliche Kaninchen vom Tierstamm HsdHHL

Watanables mit einem Körpergewicht von 3-5 kg, die zuchtbedingt unter Hypercholesterinämie und Arteriosklerose leiden, mit einem selektiven PDEV-Hemmer behandelt (3 mg/kg, intravenös).

Die Tiere hatten freien Zugang zu Wasser und konnten 2 Stunden pro Tag Futter zu sich nehmen. Die Tiere wurden in einem 10/14-Stunden Tag-Nacht-Rhythmus gehalten (Licht an ab 8.00 Uhr), und die Umgebungstemperatur betrug 22 bis 24°C.

Als selektiver PDEV-Hemmer wurde das 2-[2-Ethoxy-5-(4-ethyl-piperazin-1-sulfonyl)-phenyl]-5-methyl-7-propyl-3H-imidazo[5,1-f][1,2,4]triazin-4-on-hydrochlorid-trihydrat (Bsp. 336 der WO-A-99/24433) verwendet.

Für die intravenöse Injektion wurde der PDE-Inhibitor in physiologischer Kochsalzlösung (0,9 Vol.-%) gelöst.

Sechs der zwölf Kaninchen war 7 Tage lang vor der Behandlung mit dem PDE-Hemmer täglich, d.h. also 7 Tage aufeinanderfolgend, subkutan ein Lipidsenker, nämlich Cerivastatinnatrium (d.h. das Mononatriumsalz des Cerivastatins), verabreicht worden (1 mg/kg Körpergewicht, Cerivastatinnatrium gelöst in physiologischer Kochsalzlösung).

Den anderen sechs Kaninchen war stattdessen subkutan eine physiologische Kochsalzlösung verabreicht worden (Kontrolle).

Die sechs mit Cerivastatinnatrium vorbehandelten Kaninchen erhielten den PDE-Hemmer jeweils 1 Tag und 3 Tage nach Beendigung der 7-tägigen Cerivastatinnatriumvorbehandlung.

Die übrigen sechs, nicht mit Cerivastatinnatrium vorbehandelten Kaninchen erhielten den PDE-Hemmer 1 Tag nach Beendingung der 7tägigen subkutanen Gabe der Kochsalzlösung (Kontrolle).

Die Erektion wurde gewertet, indem die Länge des hervortretenden Penis mit einer Schieblehre gemessen wurde. Die Messung wurde jeweils 5, 10, 15, 30, 45, 60 und 120 Minuten nach Verabreichung des PDE-Hemmers durchgeführt. Die Tiere wurden dazu jedesmal aus dem Käfig geholt, am Nackenfell und den Hinterläufen festgehalten, auf den Rücken gedreht und vermessen. Unter Ruhebedingungen, d.h. im nichterigierten Zustand, ist der Kaninchenpenis in der Schamregion nicht sichtbar und von der Penishaut vollständig bedeckt.

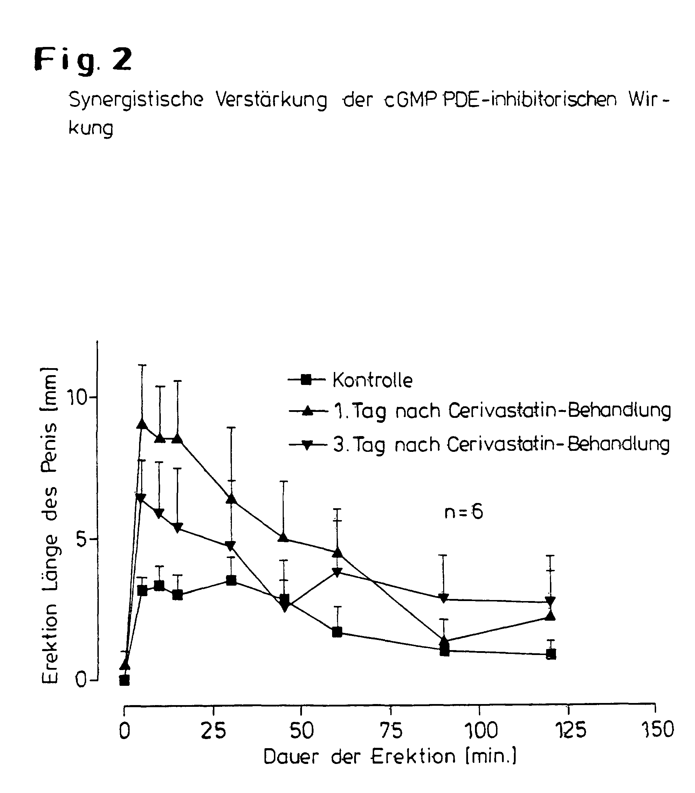

Wie die beiliegende **Fig. 2** veranschaulicht, zeigten die sechs mit dem Lipidsenker Cerivastatinnatrium vorbehandelten Kaninchen (obere und mittlere Kurven) nach Gabe des PDE-Hemmers überraschenderweise eine deutlich stärkere Erektion als die sechs Kaninchen, welche nicht mit dem Lipidsenker vorbehandelt worden waren (untere Kurve).

Ein Vergleich der oberen und mittleren Kurve der **Fig. 2** zeigt auch, dass die Verbesserung der Erektionsfähigkeit in den vorbehandelten Tieren nach Aussetzen der Behandlung mit dem Lipidsenker wieder abnahm. So war die Erektion 1 Tag nach Beendigung der Cerivastatinnatrium-Vorbehandlung noch stärker als bereits 3 Tage nach Beendigung der Cerivastatinnatrium-Vorbehandlung. Auch dies belegt, dass die überraschend eintretende Verbesserung der Wirkung des PDE-Hemmers ursächlich auf den Lipidsenker zurückzuführen ist.

Wie der Versuch zeigt, bringt bereits eine erst kurz vor Gabe der PDE-Hemmers einsetzende Vorbehandlung mit dem Lipidsenker völlig unerwartet eine Verstärkung der durch den PDE-Hemmer ausgelösten Erektion mit sich. Die auf diese Weise verbesserte Wirkung des PDE-Hemmers ist insofern überraschend, weil angesichts des kurzen Vorbehandlunsgsintervalls mit dem Lipidsenker überhaupt noch keine (signifikante) Verbesserung der Hypercholesterinämie, geschweige denn der Arteriosklerose eingetreten war.

Ohne sich auf eine bestimmte Theorie festlegen zu wollen, lässt sich die synergistische Wirkung des PDE-Hemmers zusammen mit dem Lipidsenkers möglicherweise durch eine Verbesserung der Endothelfunktion erklären, wie dies in der allgemeinen Beschreibung bereits zuvor diskutiert worden ist. Dieser synergistische Effekt war für den Fachmann aber in keiner Weise vorhersehbar und muss daher als vollkommen überraschend gewertet werden.

Das Ausführungsbeispiel belegt somit eindrucksvoll die Verbesserung der PDE-inhibitorischen Wirkung des PDE-Hemmers durch den in Kombination hiermit verabreichten Lipidsenker.

## Patentansprüche

1. Kombinationspräparat, enthaltend als pharmazeutisch wirksame Bestandteile mindestens eine Wirkstoffkomponente A und mindestens eine Wirkstoffkomponente B, **dadurch gekennzeichnet, dass** die Wirkstoffkomponente A ein PDE V-Inhibitor und die Wirkstoffkomponente B ein Lipidsenker ist.

2. Kombinationspräparat nach Anspruch 1 zur Behandlung der sexuellen Dysfunktion bei Männern oder Frauen.

3. Kombinationspräparat nach Anspruch 2 zur Behandlung der erektilen Dysfunktion.

4. Kombinationspräparat nach einem der Ansprüche 1 bis 3, **dadurch gekennzeichnet, dass** die beiden Wirkstoffkomponenten A und B entweder gleichzeitig oder aber zeitlich abgestuft angewandt werden.

**5.** Kombinationspräparat nach einem der Ansprüche 1 bis 4, **dadurch gekennzeichnet, dass** die Wirkstoffkomponenten A und B als funktionelle Einheit vorliegen, insbesondere in Form einer Mischung, eines Gemisches oder eines Gemenges.

**6.** Kombinationspräparat nach einem der Ansprüche 1 bis 4, **dadurch gekennzeichnet, dass** die Wirkstoffkomponenten A und B (räumlich) getrennt voneinander vorliegen, insbesondere als "kit-of parts".

**7.** Kombinationspräparat nach einem der Ansprüche 1 bis 6, **dadurch gekennzeichnet, dass** der Lipidsenker (Wirkstoffkomponente B) ausgewählt ist aus der Gruppe von (a) HMG-CoA-Reduktase-Inhibitoren; (b) Squalen-Synthase-Inhibitoren; (c) Gallensäure-Absorptionshemmern ("Bile acid sequestrants"); (d) Fibrinsäure und ihren Derivaten; (e) Nikotinsäure und ihren Analogen; (f) ω3-Fettsäuren.

**8.** Kombinationspräparat nach Anspruch 7, **dadurch gekennzeichnet, dass** der Lipidsenker (Wirkstoffkomponente B) ein HMG-CoA-Reduktase-Inhibitor ist und insbesondere ausgewählt ist aus der Gruppe der Statine, vorzugsweise aus der Gruppe von Atorvastatin, Cerivastatin, Fluvastatin, Lovastatin, Pravastatin, Itavastatin, Simvastatin und (+)-(3R,5S)-Bis-(7-(4-(4-fluorphenyl)-6-isopropyl-2-(N-methyl-N-methansulfonylamino)-pyrimidin-5-yl)-3,5-dihydroxy-6(E)-heptensäure, sowie deren jeweiligen Salzen, Hydraten, Alkoholaten, Estern und Tautomeren.

**9.** Kombinationspräparat nach Anspruch 8, **dadurch gekennzeichnet, dass** der Lipidsenker (Wirkstoffkomponente B) Atorvastatin oder dessen Salz, Hydrat, Alkoholat, Ester und Tautomeres ist.

**10.** Kombinationspräparat nach Anspruch 8, **dadurch gekennzeichnet, dass** der Lipidsenker (Wirkstoffkomponente B) Cerivastatin oder dessen Salz, Hydrat, Alkoholat, Ester und Tautomeres ist.

**11.** Kombinationspräparat nach einem der Ansprüche 1 bis 10, **dadurch gekennzeichnet, dass** der PDEV-Hemmer (Wirkstoffkomponente A) ausgewählt ist aus der Gruppe von Pyrazolopyrimidonen der folgenden allgemeinen Formel

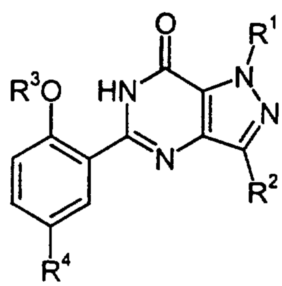

worin

$R^1$ Wasserstoff; $C_1$-$C_3$-Alkyl; $C_1$-$C_3$-Perfluoralkyl; oder $C_3$-$C_5$-Cycloalkyl bedeutet;

$R^2$ Wasserstoff; $C_1$-$C_6$-Alkyl, gegebenenfalls substituiert mit $C_3$-$C_6$-Cycloalkyl; $C_1$-$C_3$-Perfluoralkyl; oder $C_3$-$C_6$-Cycloalkyl darstellt;

$R^3$ $C_1$-$C_6$-Alkyl, gegebenenfalls substituiert mit $C_3$-$C_6$-Cycloalkyl; $C_1$-$C_6$-Perfluoralkyl, $C_3$-$C_5$-Cycloalkyl; $C_3$-$C_6$Alkenyl; oder $C_3$-$C_6$-Alkinyl ist;

$R^4$ $C_1$-$C_4$-Alkyl, gegebenenfalls substituiert mit OH, $NR^5R^6$, CN, $CONR^5R^6$ oder $CO_2R^7$; $C_2$-$C_4$-Alkenyl, gegebenenfalls substituiert mit CN, $CONR^5R^6$ oder $CO_2R^7$; $C_2$-$C_4$-Alkanoyl, gegebenenfalls substituiert mit $NR^5R^6$; (Hydroxy)-$C_2$-$C_4$-alkyl, gegebenenfalls substituiert mit $NR^5R^6$, ($C_2$-$C_3$-Alkoxy)-$C_1$-$C_2$-alkyl, gegebenenfalls substituiert mit OH oder $NR^5R^6$, $CO_2R^7$; Halogen; $NR^5R^6$, $NHSO_2NR^5R^6$; $NHSO_2R^8$; $SO_2NR^9R^{10}$; oder Phenyl, Pyridyl, Pyrimidinyl, Imidazolyl, Oxazolyl, Thiazolyl, Thienyl oder Triazolyl, von denen jedes gegebenenfalls substituiert ist mit Methyl bedeutet;

$R^5$ und $R^6$ jeweils unabhängig Wasserstoff oder $C_1$-$C_4$-Alkyl darstellen; oder zusammen mit dem Stickstoffatom, an das sie gebunden sind, eine Pyrrolidinyl-, Piperidino-, Morpholino-, 4-N($R^{11}$)-Piperazinyl- oder Imidazolyl-Gruppe bilden, wobei diese Gruppe gegebenenfalls substituiert ist mit Methyl oder OH;

$R^7$ Wasserstoff oder $C_1$-$C_4$-Alkyl ist;

$R^8$ $C_1$-$C_3$-Alkyl, gegebenenfalls substituiert mit $NR^5R^6$, bedeutet;

$R^9$ und $R^{10}$ zusammen mit dem Stickstoffatom, an das sie gebunden sind, eine Pyrrolidinyl-, Piperidino-, Morpholino-, 4-N($R^{12}$)-Piperazinyl-Gruppe bilden, wobei diese Gruppe gegebenenfalls substituiert ist mit $C_1$-$C_4$-Alkyl, $C_1$-$C_3$-Alkoxy, $NR^{13}R^{14}$ oder $CONR^{13}R^{14}$;

$R^{11}$ Wasserstoff, $C_1$-$C_3$-Alkyl, gegebenenfalls substituiert mit Phenyl; (Hydroxy)-$C_2$-$C_3$-alkyl; oder $C_1$-$C_4$-Alkanoyl darstellt;

$R^{12}$ Wasserstoff, $C_1$-$C_6$-Alkyl, ($C_1$-$C_3$-Alkoxy)-$C_2$-$C_6$-alkyl; (Hydroxy)-$C_2$-$C_6$-alkyl; ($R^{13}R^{14}N$)-$C_2$-$C_6$-Alkyl; ($R^{13}R^{14}NOC$)-$C_1$-$C_6$-Alkyl; $CONR^{13}R^{14}$; $CSNR^{13}R^{14}$, oder $C(NH)NR^{13}R^{14}$ ist; und

$R^{13}$ und $R^{14}$ jeweils unabhängig Wasserstoff; $C_1$-$C_4$-Alkyl; ($C_1$-$C_3$-Alkoxy)-$C_2$-$C_4$-alkyl; oder (Hydroxy)-$C_2$-$C_4$-alkyl bedeuten,

sowie ihren jeweiligen Salzen, Hydraten, Alkoholaten und Tautomeren.

**12.** Kombinationspräparat nach einem der Ansprüche 1 bis 10, **dadurch gekennzeichnet, dass** der PDEV-Hemmer (Wirkstoffkomponente A) ausgewählt ist aus der Gruppe von 2-phenylsubstituierten Imidazotriazinonen der allgemeinen Formel

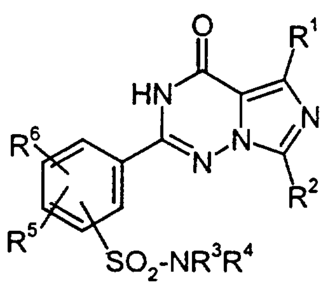

(I)

in welcher

$R^1$ für Wasserstoff oder geradkettiges oder verzweigtes Alkyl mit bis zu 4 Kohlenstoffatomen steht;

$R^2$ für geradkettiges Alkyl mit bis zu 4 Kohlenstoffatomen steht;

$R^3$ und $R^4$ gleich oder verschieden sind und für Wasserstoff oder für geradkettiges oder verzweigtes Alkenyl oder Alkoxy mit jeweils bis zu 8 Kohlenstoffatomen stehen, oder
für eine geradkettige oder verzweigte Alkylkette mit bis zu 10 Kohlenstoffatomen stehen, die gegebenenfalls durch ein Sauerstoffatom unterbrochen ist, und die gegebenenfalls ein- bis mehrfach, gleich oder verschieden durch Trifluormethyl, Trifluormethoxy, Hydroxy, Halogen, Carboxyl, Benzyloxycarbonyl, geradkettiges oder verzweigtes Alkoxycarbonyl mit bis zu 6 Kohlenstoffatomen und/oder durch Reste der Formeln $-SO_3H$, $-(A)_a-NR^7R^8$, $-O-CO-NR^{7'}R^{8'}$, $-S(O)_b-R^9$, $-P(O)(OR^{10})(OR^{11})$,

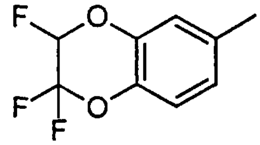
,
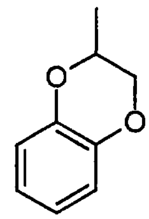
,

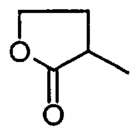
und/oder
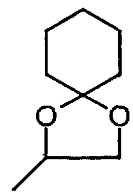

substituiert ist,
worin

a und b — gleich oder verschieden sind und eine Zahl 0 oder 1 bedeuten,

A — einen Rest CO oder $SO_2$ bedeutet,

$R^7$, $R^{7'}$, $R^8$ und $R^{8'}$ — gleich oder verschieden sind und Wasserstoff bedeuten, oder
Cycloalkyl mit 3 bis 8 Kohlenstoffatomen, Aryl mit 6 bis 10 Kohlenstoffatomen, einen 5- bis 6-gliedrigen ungesättigten, partiell ungesättigten oder gesättigten, gegebenenfalls benzokondensierten Heterocyclus, mit bis zu 3 Heteroatomen aus der Reihe S, N und/oder O bedeuten, wobei die oben aufgeführten Ringsysteme gegebenenfalls ein- bis mehrfach, gleich oder verschieden durch Hydroxy, Nitro, Trifluormethyl, Trifluormethoxy, Carboxyl, Halogen, geradkettiges oder verzweigtes Alkoxy oder Alkoxycarbonyl mit jeweils bis zu 6 Kohlenstoffatomen oder durch eine Gruppe der Formel $-(SO_2)_c-NR^{12}R^{13}$ substituiert sind,
worin

c — eine Zahl 0 oder 1 bedeutet,

$R^{12}$ und $R^{13}$ — gleich oder verschieden sind und Wasserstoff oder geradkettiges oder verzweigtes Alkyl mit bis zu 5 Kohlenstoffatomen bedeuten,

oder

$R^7$, $R^{7'}$, $R^8$ und $R^{8'}$ — geradkettiges oder verzweigtes Alkoxy mit bis zu 6 Kohlenstoffatomen bedeuten, oder
geradkettiges oder verzweigtes Alkyl mit bis zu 8 Kohlenstoffatomen bedeuten, das gegebenenfalls ein- oder mehrfach, gleich oder verschieden durch Hydroxy, Halogen, Aryl mit 6 bis 10 Kohlenstoffatomen, geradkettiges oder verzweigtes Alkoxy oder Alkoxycarbonyl mit jeweils bis zu 6 Kohlenstoffatomen oder durch eine Gruppe der Formel $-(CO)_d-NR^{14}R^{15}$ substituiert ist,
worin

$R^{14}$ und $R^{15}$ — gleich oder verschieden sind und Wasserstoff oder geradkettiges oder verzweigtes Alkyl mit bis zu 4 Kohlenstoffatomen bedeuten,

und

d eine Zahl 0 oder 1 bedeutet,

oder

$R^7$ und $R^8$ und/oder $R^{7'}$ und $R^{8'}$ gemeinsam mit dem Stickstoffatom einen 5-bis 7-gliedrigen, gesättigten Heterocyclus bilden, der gegebenenfalls noch ein weiteres Heteroatom aus der Reihe S oder O oder einen Rest der Formel $-NR^{16}$ enthalten kann, worin

$R^{16}$ Wasserstoff, Aryl mit 6 bis 10 Kohlenstoffatomen, Benzyl, einen 5- bis 7-gliedrigen aromatischen oder gesättigten Heterocyclus mit bis zu 3 Heteroatomen aus der Reihe S, N und/oder O bedeutet, der gegebenenfalls durch Methyl substituiert ist, oder geradkettiges oder verzweigtes Alkyl mit bis zu 6 Kohlenstoffatomen bedeutet, das gegebenenfalls durch Hydroxy substituiert ist,

$R^9$ Aryl mit 6 bis 10 Kohlenstoffatomen bedeutet, oder geradkettiges oder verzweigtes Alkyl mit bis zu 4 Kohlenstoffatomen bedeutet,

$R^{10}$ und $R^{11}$ gleich oder verschieden sind und Wasserstoff oder geradkettiges oder verzweigtes Alkyl mit bis zu 4 Kohlenstoffatomen bedeuten, und/oder die oben unter $R^3/R^4$ aufgeführte Alkylkette gegebenenfalls durch Cycloalkyl mit 3 bis 8 Kohlenstoffatomen, Aryl mit 6 bis 10 Kohlenstoffatomen oder durch einen 5- bis 7-gliedrigen, partiell ungesättigten, gesättigten oder ungesättigten, gegebenenfalls benzokondensierten Heterocyclus, der bis zu 4 Heteroatome aus der Reihe S, N; O oder einen Rest der Formel $-NR^{17}$ enthalten kann, substituiert ist, worin

$R^{17}$ Wasserstoff, Hydroxy, Formyl, Trifluormethyl, geradkettiges oder verzweigtes Acyl oder Alkoxy mit jeweils bis zu 4 Kohlenstoffatomen bedeutet, oder geradkettiges oder verzweigtes Alkyl mit bis zu 6 Kohlenstoffatomen bedeutet, das gegebenenfalls ein- bis mehrfach, gleich oder verschieden durch Hydroxy, oder geradkettiges oder verzweigtes Alkoxy mit bis zu 6 Kohlenstoffatomen substituiert ist, und wobei Aryl und der Heterocyclus gegebenenfalls ein- bis mehrfach, gleich oder verschieden durch Nitro, Halogen, $-SO_3H$, geradkettiges oder verzweigtes Alkyl oder Alkoxy mit jeweils bis zu 6 Kohlenstoffatomen, Hydroxy, Trifluormethyl, Trifluormethoxy und/oder durch einen Rest der Formel $-SO_2NR^{18}R^{19}$ substituiert sind, worin

$R^{18}$ und $R^{19}$ gleich oder verschieden sind und Wasserstoff oder geradkettiges oder verzweigtes Alkyl mit bis zu 6 Kohlenstoffatomen bedeuten, und/oder

$R^3$ oder $R^4$ für eine Gruppe der Formel $-NR^{20}R^{21}$ steht, worin

$R^{20}$ und $R^{21}$ die oben angegebene Bedeutung von $R^{18}$ und $R^{19}$ haben und mit dieser gleich oder verschieden sind, und/oder

$R^3$ oder $R^4$ für Adamantyl stehen, oder für Reste der Formeln

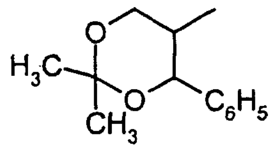
,
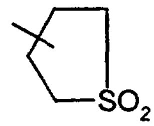
,
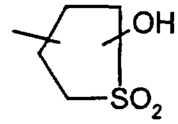

oder

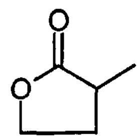

stehen,
oder für Cycloalkyl mit 3 bis 8 Kohlenstoffatomen, Aryl mit 6 bis 10 Kohlenstoffatomen oder für einen 5- bis 7-gliedrigen partiell ungesättigten, gesättigten oder ungesättigten, gegebenenfalls benzokondensierten Heterocyclus stehen, der bis zu 4 Heteroatome aus der Reihe S, N; O oder einen Rest der Formel $-NR^{22}$ enthalten kann,

worin

$R^{22}$ die oben angegebene Bedeutung von $R^{16}$ hat und mit dieser gleich oder verschieden ist, oder Carboxyl, Formyl oder geradkettiges oder verzweigtes Acyl mit bis zu 5 Kohlenstoffatomen bedeutet,
und wobei Cycloalkyl, Aryl und/oder der Heterocyclus gegebenenfaHs ein- bis mehrfach, gleich oder verschieden durch Halogen, Triazolyl, Trifluormethyl, Trifluormethoxy, Carboxyl, geradkettiges oder verzweigtes Acyl oder Alkoxycarbonyl mit jeweils bis zu 6 Kohlenstoffatomen, Nitro und/ oder durch Gruppen der Formeln $-SO_3H$, $-OR^{23}$, $(SO_2)_eNR^{24}R^{25}$, $-P(O)(OR^{26})(OR^{27})$ substituiert sind,
worin

e eine Zahl 0 oder 1 bedeutet,

$R^{23}$ einen Rest der Formel

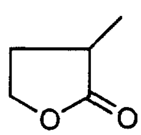

bedeutet, oder
Cycloalkyl mit 3 bis 7 Kohlenstoffatomen bedeutet, oder Wasserstoff oder geradkettiges oder verzweigtes Alkyl mit bis zu 4 Kohlenstoffatomen bedeutet, das gegebenenfalls durch Cycloalkyl mit 3 bis 7 Kohlenstoffatomen, Benzyloxy, Tetrahydropyranyl, Tetrahydrofuranyl, geradkettiges oder verzweigtes Alkoxy oder Alkoxycarbonyl mit jeweils bis zu 6 Kohlenstoffatomen, Carboxyl, Benzyloxycarbonyl oder Phenyl substituiert ist, das seinerseits ein- bis mehrfach, gleich oder verschieden durch geradkettiges oder verzweigtes Alkoxy mit bis zu 4 Kohlenstoffatomen, Hydroxy oder Halogen substituiert sein kann,
und/oder Alkyl gegebenenfalls durch Reste der Formeln $-CO-NR^{28}R^{29}$ oder $-CO-R^{30}$ substituiert

ist,
worin

$R^{28}$ und $R^{29}$ gleich oder verschieden sind und Wasserstoff oder geradkettiges oder verzweigtes Alkyl mit bis zu 8 Kohlenstoffatomen bedeuten, oder

$R^{28}$ und $R^{29}$ gemeinsam mit dem Stickstoffatom einen 5- bis 7-gliedrigen gesättigten Heterocyclus bilden, der gegebenenfalls ein weiteres Heteroatom aus der Reihe S oder O enthalten kann,
und

$R^{30}$ Phenyl oder Adamantyl bedeutet,

$R^{24}$ und $R^{25}$ die oben angegebene Bedeutung von $R^{18}$ und $R^{19}$ haben und mit dieser gleich oder verschieden sind,

$R^{26}$ und $R^{27}$ die oben angegebene Bedeutung von $R^{10}$ und $R^{11}$ haben und mit dieser gleich oder verschieden sind

und/oder Cycloalkyl, Aryl und/oder der Heterocyclus gegebenenfalls durch geradkettiges oder verzweigtes Alkyl mit bis zu 6 Kohlenstoffatomen substituiert sind, das gegebenenfalls durch Hydroxy, Carboxyl, durch einen 5-bis 7-gliedrigen Heterocyclus mit bis zu 3 Heteroatomen aus der Reihe S, N und/oder O oder durch Gruppen der Formel $-SO_2-R^{31}$, $P(O)(OR^{32})(OR^{33})$ oder $-NR^{34}R^{35}$ substituiert ist,
worin

$R^{31}$ Wasserstoff bedeutet oder die oben angegebene Bedeutung von $R^9$ hat und mit dieser gleich oder verschieden ist,

$R^{32}$ und $R^{33}$ die oben angegebene Bedeutung von $R^{10}$ und $R^{11}$ haben und mit dieser gleich oder verschieden sind,

$R^{34}$ und $R^{35}$ gleich oder verschieden sind und Wasserstoff oder geradkettiges oder verzweigtes Alkyl mit bis zu 6 Kohlenstoffatomen bedeuten, das gegebenenfalls durch Hydroxy oder geradkettiges oder verzweigtes Alkoxy mit bis zu 4 Kohlenstoffatomen substituiert ist, oder

$R^{34}$ und $R^{35}$ gemeinsam mit dem Stickstoffatom einen 5- bis 6-gliedrigen gesättigten Heterocyclus bilden, der ein weiteres Heteroatom aus der Reihe S oder O oder einen Rest der Formel $-NR^{36}$ enthalten kann, worin

$R^{36}$ Wasserstoff, Hydroxy, geradkettiges oder verzweigtes Alkoxycarbonyl mit bis zu 7 Kohlenstoffatomen oder geradkettiges oder verzweigtes Alkyl mit bis zu 5 Kohlenstoffatomen bedeutet, das gegebenenfalls durch Hydroxy substituiert ist,

oder

$R^3$ und $R^4$ gemeinsam mit dem Stickstoffatom einen 5- bis 7-gliedrigen, ungesättigten oder gesättigten oder partiell ungesättigten, gegebenenfalls benzokondensierten Heterocyclus bilden, der gegebenenfalls bis zu 3 Heteroatome aus der Reihe S, N, O oder einen Rest der Formel $-NR^{37}$ enthalten kann,

worin

$R^{37}$ Wasserstoff, Hydroxy, Formyl, Trifluormethyl, geradkettiges oder verzweigtes Acyl, Alkoxy oder Alkoxycarbonyl mit jeweils bis zu 4 Kohlenstoffatomen bedeutet,
oder geradkettiges oder verzweigtes Alkyl mit bis zu 6 Kohlenstoffatomen bedeutet, das gegebenenfalls ein- bis mehrfach, gleich oder verschieden durch Hydroxy, Trifluormethyl, Carboxyl, geradkettiges oder verzweigtes Alkoxy oder Alkoxycarbonyl mit jeweils bis zu 6 Kohlenstoffatomen oder durch Gruppen der Formel $-(D)_f-NR^{38}R^{39}$, $-CO-(CH_2)_g-O-CO-R^{40}$, $-CO-(CH_2)_h-OR^{41}$ oder $-P(O)(OR^{42})(OR^{43})$ substituiert ist,

worin

g und h gleich oder verschieden sind und eine Zahl 1, 2, 3 oder 4 bedeuten, und

f eine Zahl 0 oder 1 bedeutet,

D eine Gruppe der Formel -CO oder $-SO_2$ bedeutet,

$R^{38}$ und $R^{39}$ gleich oder verschieden sind und die oben angegebene Bedeutung von $R^7$ und $R^8$ haben,

$R^{40}$ geradkettiges oder verzweigtes Alkyl mit bis zu 6 Kohlenstoffatomen bedeutet,

$R^{41}$ geradkettiges oder verzweigtes Alkyl mit bis zu 6 Kohlenstoffatomen bedeutet,

$R^{42}$ und $R^{43}$ gleich oder verschieden sind und Wasserstoff oder geradkettiges oder verzweigtes Alkyl mit bis zu 4 Kohlenstoffatomen bedeuten,

oder

$R^{37}$ einen Rest der Formel $-(CO)_i-E$ bedeutet, worin

i eine Zahl 0 oder 1 bedeutet,

E Cycloalkyl mit 3 bis 7 Kohlenstoffatomen oder Benzyl bedeutet, Aryl mit 6 bis 10 Kohlenstoffatomen oder einen 5- bis 6-gliedrigen aromatischen Heterocyclus mit bis zu 4 Heteroatomen aus der Reihe S, N und/oder O bedeutet, wobei die oben aufgeführten Ringsysteme gegebenenfalls ein- bis mehrfach, gleich oder verschieden durch Nitro, Halogen, $-SO_3H$, geradkettiges oder verzweigtes Alkoxy mit bis zu 6 Kohlenstoffatomen, Hydroxy, Trifluormethyl, Trifluormethoxy oder durch einen Rest der Formel $-SO_2-NR^{44}R^{45}$, substituiert sind, worin

$R^{44}$ und $R^{45}$ die oben angegebene Bedeutung von $R^{18}$ und $R^{19}$ haben und mit dieser gleich oder verschieden sind,

oder

E Reste der Formeln

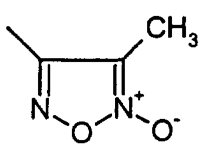

,

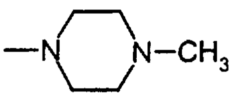

oder

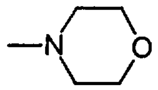

bedeutet,

und der unter $R^3$ und $R^4$ aufgeführte, gemeinsam mit dem Stickstoffatom gebildete Heterocyclus, gegebenenfalls ein- bis mehrfach, gleich oder verschieden, gegebenenfalls auch geminal, durch Hydroxy, Formyl, Carboxyl, geradkettiges oder verzweigtes Acyl oder Alkoxycarbonyl mit bis jeweils zu 6 Kohlenstoffatomen, Nitro und Gruppen der Formeln $-P(O)(OR^{46})(OR^{47})$,

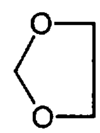 ,

$$=NR^{48} \text{ oder } —(CO)_jNR^{49}R^{50}$$

substituiert ist,
worin

$R^{46}$ und $R^{47}$ die oben angegebene Bedeutung von $R^{10}$ und $R^{11}$ haben und mit dieser gleich oder verschieden sind,

$R^{48}$ Hydroxy oder geradkettiges oder verzweigtes Alkoxy mit bis zu 4 Kohlenstoffatomen bedeutet,

j eine Zahl 0 oder 1 bedeutet,
und

$R^{49}$ und $R^{50}$ gleich oder verschieden sind und die oben angegebene Bedeutung von $R^{14}$ und $R^{15}$ haben,

und/oder der unter $R^3$ und $R^4$ aufgeführte, gemeinsam mit dem Stickstoffatom gebildete Heterocyclus, gegebenenfalls durch geradkettiges oder verzweigtes Alkyl mit bis zu 6 Kohlenstoffatomen substituiert ist, das gegebenenfalls einbis mehrfach, gleich oder verschieden durch Hydroxy, Halogen, Carboxyl, Cycloalkyl oder Cycloalkyloxy mit jeweils 3 bis 8 Kohlenstoffatomen, geradkettiges oder verzweigtes Alkoxy oder Alkoxycarbonyl mit jeweils bis zu 6 Kohlenstoffatomen oder durch einen Rest der Formel $-SO_3H$, $-NR^{51}R^{52}$ oder $P(O)OR^{53}OR^{54}$ substituiert ist,
worin

$R^{51}$ und $R^{52}$ gleich oder verschieden sind und Wasserstoff, Phenyl, Carboxyl, Benzyl oder geradkettiges oder verzweigtes Alkyl oder Alkoxy mit jeweils bis zu 6 Kohlenstoffatomen bedeuten,

$R^{53}$ und $R^{54}$ gleich oder verschieden sind und die oben angegebene Bedeutung von $R^{10}$ und $R^{11}$ haben,

und/oder das Alkyl gegebenenfalls durch Aryl mit 6 bis 10 Kohlenstoffatomen substituiert ist, das seinerseits einbis mehrfach, gleich oder verschieden durch Halogen, Hydroxy, geradkettiges oder verzweigtes Alkoxy mit bis zu 6 Kohlenstoffatomen, oder durch eine Gruppe der Formel $-NR^{51'}R^{52'}$ substituiert sein kann,
worin

$R^{51'}$ und $R^{52'}$ die oben angegebene Bedeutung von $R^{51}$ und $R^{52}$ haben und mit dieser gleich oder verschieden

sind,

und/oder der unter $R^3$ und $R^4$ aufgeführte, gemeinsam mit dem Stickstoffatom gebildete Heterocyclus, gegebenenfalls durch Aryl mit 6 bis 10 Kohlenstoffatomen oder durch einen 5- bis 7-gliedrigen, gesättigen, partiell ungesättigten oder ungesättigten Heterocyclus mit bis zu 3 Heteroatomen aus der Reihe S, N und/oder O , gegebenenfalls auch über eine N-Funktion verknüpft, substituiert ist, wobei die Ringsysteme ihrerseits durch Hydroxy oder durch geradkettiges oder verzweigtes Alkyl oder Alkoxy mit jeweils bis zu 6 Kohlenstoffatomen substituiert sein können,
oder

$R^3$ und $R^4$ gemeinsam mit dem Stickstoffatom Reste der Formeln

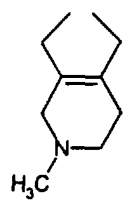
,
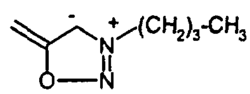
,
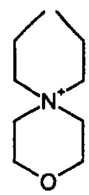

oder

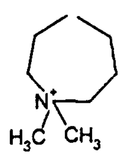

bilden,

$R^5$ und $R^6$ gleich oder verschieden sind und für Wasserstoff, geradkettiges oder verzweigtes Alkyl mit bis zu 6 Kohlenstoffatomen, Hydroxy oder für geradkettiges oder verzweigtes Alkoxy mit bis zu 6 Kohlenstoffatomen stehen.

sowie ihren jeweiligen Salzen, Hydraten, Alkoholaten und Tautomeren.

**13.** Kombinationspräparat nach einem der Ansprüche 1 bis 10, **dadurch gekennzeichnet, dass** der PDEV-Hemmer (Wirkstoffkomponente A) ausgewählt ist aus der Gruppe von (a) 5-[2-Ethoxy-5-(4-methyl-1-piperazinylsulfonyl)-phenyl]-1-methyl-3-n-propyl-1,6-dihydro-7H-pyrazolo-[4,3-d]-pyrimidin-7-on (Sildenafil) sowie dessen Salzen, Hydraten, Alkoholaten und Tautomeren; und (b) 2-[2-Ethoxy-5-(4-ethyl-piperazin-1-sulfonyl)-phenyl]-5-methyl-7-propyl-3H-imidazo[5,1-f]-[1,2,4]-triazin-4-on sowie dessen Salzen, Hydraten, Alkoholaten und Tautomeren.

**14.** Kombinationspräparat nach Anspruch 13, dadurch dass der PDEV-Hemmer (Wirkstoffkomponente A) das S-[2-Ethoxy-S-(4-methyl-1-piperazinylsulfonyl)-phenyl]-1-methyl-3-n-propyl-1,6-dihydro-7H-pyrazolo-[4,3-d]-pyrimidin-7-on-citrat (Sildenafilcitrat, Viagra™) oder das 2-[2-Ethoxy-5-(4-ethylpiperazin-1-sulfonyl)-phenyl]-5-methyl-7-propyl-3H-imidazo[5,1-f][1,2,4]triazin-4-on-hydrochlorid-trihydrat ist.

**15.** Verwendung von Lipidsenkern zur Steigerung der Wirksamkeit von PDEV-Inhibitoren.

**16.** Verwendung nach Anspruch 15 zur Herstellung eines Arzneimittels zur Behandlung der sexuellen Dysfunktion bei Männern und Frauen, insbesondere bei der Behandlung der erektilen Dysfunktion.

**17.** Verwendung nach Anspruch 15 oder 16, **dadurch gekennzeichnet, dass** der Lipidsenker und der PDEV-Inhibitor entweder gleichzeitig oder aber zeitlich abgestuft angewandt werden.

**18.** Verwendung nach einem der Ansprüche 15 bis 17, **dadurch gekennzeichnet, dass** der Lipidsenker und der PDEV-Hemmer als funktionelle Einheit vorliegen, insbesondere in Form einer Mischung, eines Gemisches oder eines Gemenges.

**19.** Verwendung nach einem der Ansprüche 15 bis 17, **dadurch gekennzeichnet, dass** der Lipidsenker und der PDEV-Hemmer (räumlich) getrennt voneinander vorliegen, insbesondere als "kit-of-parts".

**20.** Verwendung nach einem der Ansprüche 15 bis 19, **dadurch gekennzeichnet, dass** der Lipidsenker aus den in den Ansprüchen 7 bis 10 definierten Verbindungen ausgewählt ist.

**21.** Verwendung nach einem der Ansprüche 15 bis 20, **dadurch gekennzeichnet, dass** der PDEV-Hemmer aus den in den Ansprüchen 11 bis 14 definierten Verbindungen ausgewählt ist.

## Claims

**1.** Combination preparation comprising as pharmaceutically active ingredients at least one active compound component A and at least one active compound component B, **characterized in that** the active compound component A is a PDE V inhibitor and the active compound component B is an antilipemic.

**2.** Combination preparation according to Claim 1 for treating sexual dysfunction in men or women.

**3.** Combination preparation according to Claim 2 for treating erectile dysfunction.

**4.** Combination preparation according to any of Claims 1 to 3, **characterized in that** the two active compound components A and B are used either simultaneously or successively.

**5.** Combination preparation according to any of Claims 1 to 4, **characterized in that** the active compound components A and B are present as a functional unit, in particular in the form of a mixture, a mix or a blend.

**6.** Combination preparation according to any of Claims 1 to 4, **characterized in that** the active compound components A and B are (spatially) separated, in particular as a kit-of-parts.

**7.** Combination preparation according to any of Claims 1 to 6, **characterized in that** the antilipemic (active compound component B) is selected from the group consisting of (a) HMG-CoA-reductase inhibitors; (b) squalene synthase inhibitors; (c) bile acid sequestrants; (d) fibric acid and its derivatives; (e) nicotinic acid and its analogs; (f) ω3-fatty acids.

**8.** Combination preparation according to Claim 7, **characterized in that** the antilipemic (active compound component B) is an HMG-CoA-reductase inhibitor and is in particular selected from the group of the statins, preferably from the group consisting of atorvastatin, cerivastatin, fluvastatin, lovastatin, pravastatin, itavastatin, simvastatin and (+)-(3R,5S)-bis-(7-(4-(4-fluorophenyl)-6-isopropyl-2-(N-methyl-N-methanesulfonylamino)-pyrimidin-5-yl)-3,5-dihydroxy-6(E)-heptenoic acid, and their respective salts, hydrates, alkoxides, esters and tautomers.

**9.** Combination preparation according to Claim 8, **characterized in that** the antilipemic (active compound component B) is atorvastatin or its salt, hydrate, alkoxide, ester and tautomer.

**10.** Combination preparation according to Claim 8, **characterized in that** the antilipemic (active compound component B) is cerivastatin or its salt, hydrate, alkoxide, ester and tautomer.

**11.** Combination preparation according to any of Claims 1 to 10, **characterized in that** the PDE V inhibitor (active compound component A) is selected from the group consisting ofpyrazolopyrimidones of the general formula below

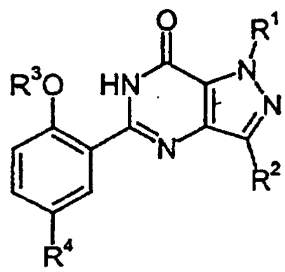

in which

| | |
|---|---|
| $R^1$ | represents hydrogen; $C_1$-$C_3$-alkyl; $C_1$-$C_3$-perfluoroalkyl; or $C_3$-$C_5$-cycloalkyl; |
| $R^2$ | denotes hydrogen; $C_1$-$C_6$-alkyl, optionally substituted by $C_3$-$C_6$-cycloalkyl; $C_1$-$C_3$-perfluoroalkyl; or $C_3$-$C_6$-cycloalkyl; |
| $R^3$ | is $C_1$-$C_6$-alkyl, optionally substituted by $C_3$-$C_6$-cycloalkyl; $C_1$-$C_6$-perfluoroalkyl, $C_3$-$C_5$-cycloalkyl; $C_3$-$C_6$alkenyl; or $C_3$-$C_6$-alkinyl; |
| $R^4$ | represents $C_1$-$C_4$-alkyl, optionally substituted by OR, $NR^5R^6$, CN, $CONR^5R^6$ or $CO_2R^7$; $C_2$-$C_4$-alkenyl, optionally substituted by CN, $CONR^5R^6$ or $CO_2R^7$; $C_2$-$C_4$-alkanoyl, optionally substituted by $NR^5R^6$; (hydroxy)-$C_2$-$C_4$-alkyl, optionally substituted by $NR^5R^6$, ($C_2$-$C_3$-alkoxy)-$C_1$-$C_2$-alkyl, optionally substituted by OH or $NR^5R^6$, $CO_2R^7$; halogen; $NR^5R^6$, $NHSO_2NR^5R^6$; $NHSO_2R^8$; $SO_2NR^9R^{10}$ or phenyl, pyridyl, pyrimidinyl, imidazolyl, oxazolyl, thiazolyl, thienyl or triazolyl, each of which is optionally substituted by methyl; |
| $R^5$ and $R^6$ | each independently of one another denote hydrogen or $C_1$-$C_4$-alkyl; or together with the nitrogen atom to which they are attached form a pyrrolidinyl, piperidino, morpholino, 4-N($R^{11}$)-piperazinyl or imidazolyl group, where this group is optionally substituted by methyl or OH; |
| $R^7$ | is hydrogen or $C_1$-$C_4$-alkyl; |
| $R^8$ | represents $C_1$-$C_3$-alkyl, optionally substituted by $NR^5R^6$; |
| $R^9$ and $R^{10}$ | together with the nitrogen atom to which they are attached form a pyrrolidinyl, piperidino, morpholino, 4-N($R^{12}$)-piperazinyl group, where this group is optionally substituted by $C_1$-$C_4$-alkyl, $C_1$-$C_3$-alkoxy, $NR^{13}R^{14}$ or $CONR^{13}R^{14}$; |
| $R^{11}$ | denotes hydrogen, $C_1$-$C_3$-alkyl, optionally substituted by phenyl; (hydroxy)-$C_2$-$C_3$-alkyl; or $C_1$-$C_4$-alkanoyl; |
| $R^{12}$ | is hydrogen, $C_1$-$C_6$-alkyl, ($C_1$-$C_3$-alkoxy)-$C_2$-$C_6$-alkyl; (hydroxy)-$C_2$-$C_6$-alkyl; ($R^{13}R^{14}N$-$C_2$-$C_6$-alkyl; ($R^{13}R^{14}NOC$)-$C_1$-$C_6$-alkyl; $CONR^{13}R^{14}$; $CSNR^{13}R^{14}$, or $C(NH)NR^{13}R^{14}$; and |
| $R^{13}$ and $R^{14}$ | each independently of one another represent hydrogen; $C_1$-$C_4$-alkyl; ($C_1$-$C_3$-alkoxy)-$C_2$-$C_4$-alkyl; or (hydroxy)-$C_2$-$C_4$-alkyl, |

and their respective salts, hydrates, alkoxides and tautomers.

**12.** Combination preparation according to any of Claims 1 to 10, **characterized in that** the PDE V inhibitor (active compound component A) is selected from the group consisting of 2-phenyl-substituted imidazotriazinones of the general formula

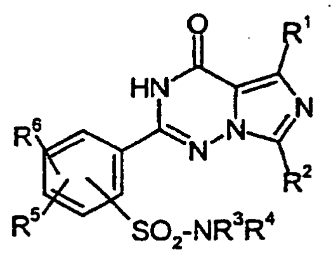 (I)

in which

| | |
|---|---|
| $R^1$ | represents hydrogen or straight-chain or branched alkyl having up to 4 carbon atoms; |
| $R^2$ | represents straight-chain alkyl having up to 4 carbon atoms; |
| $R^3$ and $R^4$ | are identical or different and represent hydrogen or represent straight-chain or branched alkenyl or alkoxy having in each case up to 8 carbon atoms, or represent a straight-chain or branched alkyl chain having up to 10 carbon atoms which is optionally interrupted by an oxygen atom and which is optionally mono- to polysubstituted by identical or different substituents from the group consisting of trifluoromethyl, trifluoromethoxy, hydroxyl, halogen, carboxyl, benzyloxycarbonyl, straight-chain or branched alkoxycarbonyl having up to 6 carbon atoms and/or by radicals of the formulae $-SO_3H$, $-(A)_a-NR^7R^8$, $-O-CO-NR^{7'}R^{8'}$, $-S(O)_b-R^9$, $-P(O)(OR^{10})(OR^{11})$, |

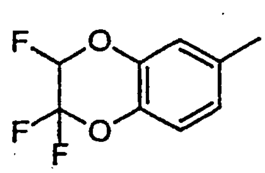 , 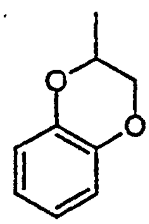 ,

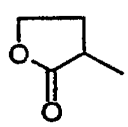 and/or 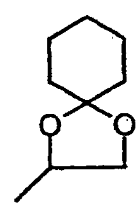

in which

| | |
|---|---|
| a and b | are identical or different and represent a number 0 or 1, |
| A | represents a radical CO or $SO_2$, |
| $R^7$, $R^{7'}$, $R^8$ and $R^{8'}$ | are identical or different and represent hydrogen, or represent cycloalkyl having 3 to 8 carbon atoms, aryl having 6 to 10 carbon atoms, a 5- to 6-membered un- |

saturated, partially unsaturated or saturated optionally benzo-fused heterocycle having up to 3 heteroatoms from the group consisting of S, N and O, where the abovementioned ring systems are optionally mono- to polysubstituted by identical or different substituents from the group consisting of hydroxyl, nitro, trifluoromethyl, trifluoromethoxy, carboxyl, halogen, straight-chain or branched alkoxy or alkoxycarbonyl having in each case up to 6 carbon atoms or by a group of the formula $-(SO_2)_c-NR^{12}R^{13}$,
in which

c represents a number 0 or 1,

$R^{12}$ and $R^{13}$ are identical or different and represent hydrogen or straight-chain or branched alkyl having up to 5 carbon atoms,

or

$R^7$, $R^{7'}$, $R^8$ and $R^{8'}$ represent straight-chain or branched alkoxy having up to 6 carbon atoms, or represent straight-chain or branched alkyl having up to 8 carbon atoms which is optionally mono- or polysubstituted by identical or different substituents from the group consisting of hydroxyl, halogen, aryl having 6 to 10 carbon atoms, straight-chain or branched alkoxy or alkoxycarbonyl having in each case up to 6 carbon atoms, or by a group of the formula $-(CO)_d-NR^{14}R^{15}$,
in which

$R^{14}$ and $R^{15}$ are identical or different and represent hydrogen or straight-chain or branched alkyl having up to 4 carbon atoms,

and

d represents a number 0 or 1,

or

$R^7$ and $R^8$ and/or $R^{7'}$ and $R^{8'}$ together with the nitrogen atom form a 5- to 7-membered saturated heterocycle which may optionally contain a further heteroatom from the group consisting of S and O or a radical of the formula $-NR^{16}$,
in which

$R^{16}$ represents hydrogen, aryl having 6 to 10 carbon atoms, benzyl, a 5- to 7-membered aromatic or saturated heterocycle having up to 3 heteroatoms from the group consisting of S, N and O, which heterocycle is optionally substituted by methyl, or represents straight-chain or branched alkyl having up to 6 carbon atoms which is optionally substituted by hydroxyl,

$R^9$ represents aryl having 6 to 10 carbon atoms, or represents straight-chain or branched alkyl having up to 4 carbon atoms,

$R^{10}$ and $R^{11}$ are identical or different and represent hydrogen or straight-chain or branched alkyl having up to 4 carbon atoms,

and/or the alkyl chain listed above under $R^3$/$R^4$ is optionally substituted by cycloalkyl having 3 to 8 carbon atoms, aryl having 6 to 10 carbon atoms or by a 5- to 7-membered partially unsaturated, saturated or unsaturated optionally benzo-fused heterocycle which may contain up to 4 heteroatoms from the group consisting of S, N; O or a radical of the formula $-NR^{17}$,
in which

$R^{17}$ represents hydrogen, hydroxyl, formyl, trifluoromethyl, straight-chain or branched acyl or alkoxy having in

each case up to 4 carbon atoms, or represents straight-chain or branched alkyl having up to 6 carbon atoms which is optionally mono- to polysubstituted by identical or different substituents from the group consisting of hydroxyl and straight-chain or branched alkoxy having up to 6 carbon atoms,

and where aryl and the heterocycle are optionally mono- to polysubstituted by identical or different substituents from the group consisting of nitro, halogen, $-SO_3H$, straight-chain or branched alkyl or alkoxy having in each case up to 6 carbon atoms, hydroxyl, trifluoromethyl, trifluoromethoxy and/or by a radical of the formula $-SO_2NR^{18}R^{19}$, in which

$R^{18}$ and $R^{19}$ are identical or different and represent hydrogen or straight-chain or branched alkyl having up to 6 carbon atoms,

and/or

$R^3$ or $R^4$ represent a group of the formula $-NR^{20}R^{21}$,

in which

$R^{20}$ and $R^{21}$ have the meaning of $R^{18}$ and $R^{19}$ given above and are identical to or different from this meaning,

and/or

$R^3$ or $R^4$ represent adamantyl, or

represent radicals of the formulae

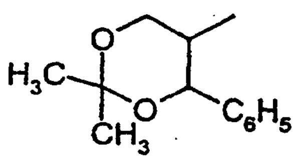 , 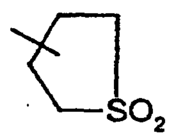 , 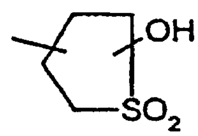

or

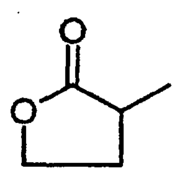 ,

or represent cycloalkyl having 3 to 8 carbon atoms, aryl having 6 to 10 carbon atoms or represent a 5- to 7-membered partially unsaturated, saturated or unsaturated optionally benzo-fused heterocycle which may contain up to 4 heteroatoms from the group consisting of S, N; O or a radical of the formula $-NR^{22}$, in which

$R^{22}$ has the meaning of $R^{16}$ given above and is identical to or different from this meaning, or represents carboxyl, formyl or straight-chain or branched acyl having up to 5 carbon atoms,

and where cycloalkyl, aryl and/or the heterocycle are optionally mono- to polysubstituted by identical or different substituents from the group consisting of halogen, triazolyl, trifluoromethyl, trifluoromethoxy, carboxyl, straight-chain or branched acyl or alkoxycarbonyl having in each case up to 6 carbon atoms, nitro, and/or by groups of the formulae $-SO_3H$, $-OR^{23}$, $(SO_2)_eNR^{24}R^{25}$, $-P(O)(OR^{26})(OR^{27})$,

in which

e represents a number 0 or 1,

$R^{23}$ represents a radical of the formula

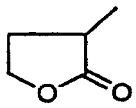,

or represents cycloalkyl having 3 to 7 carbon atoms, or
represents hydrogen or straight-chain or branched alkyl having up to 4 carbon atoms which is optionally substituted by cycloalkyl having 3 to 7 carbon atoms, benzyloxy, tetrahydropyranyl, tetrahydrofuranyl, straight-chain or branched alkoxy or alkoxycarbonyl having in each case up to 6 carbon atoms, carboxyl, benzyloxycarbonyl or phenyl which for its part may be mono- to polysubstituted by identical or different substituents from the group consisting of straight-chain or branched alkoxy having up to 4 carbon atoms, hydroxyl and halogen, and/or alkyl is optionally substituted by radicals of the formulae $-CO-NR^{28}R^{29}$ or $-CO-R^{30}$,
in which

$R^{28}$ and $R^{29}$ are identical or different and represent hydrogen or straight-chain or branched alkyl having up to 8 carbon atoms, or

$R^{28}$ and $R^{29}$ together with the nitrogen atom form a 5- to 7-membered saturated heterocycle which may optionally contain a further heteroatom from the group consisting of S and O,
and

$R^{30}$ represents phenyl or adamantyl,

$R^{24}$ and $R^{25}$ have the meaning of $R^{18}$ and $R^{19}$ given above and are identical to or different from this meaning,

$R^{26}$ and $R^{27}$ have the meaning of $R^{10}$ and $R^{11}$ given above and are identical to or different from this meaning

and/or cycloalkyl, aryl and/or the heterocycle are optionally substituted by straight-chain or branched alkyl having up to 6 carbon atoms which is optionally substituted by hydroxyl, carboxyl, by a 5- to 7-membered heterocycle having up to 3 heteroatoms from the group consisting of S, N and O or by groups of the formula -SO2-R31, P(O)$(OR^{32})(OR^{33})$ or $-NR^{34}R^{35}$,
in which

$R^{31}$ is hydrogen or has the meaning of $R^{9}$ given above and is identical to or different from this meaning,

$R^{32}$ and $R^{33}$ have the meaning of $R^{10}$ and $R^{11}$ given above and are identical to or different from this meaning,

$R^{34}$ and $R^{35}$ are identical or different and represent hydrogen or straight-chain or branched alkyl having up to 6 carbon atoms which is optionally substituted by hydroxyl or straight-chain or branched alkoxy having up to 4 carbon atoms, or

$R^{34}$ and $R^{35}$ together with the nitrogen atom form a 5- to 6-membered saturated heterocycle which may contain a further heteroatom from the group consisting of S and O or a radical of the formula $-NR^{36}$,
in which

$R^{36}$ represents hydrogen, hydroxyl, straight-chain or branched alkoxycarbonyl having up to 7 carbon atoms or straight-chain or branched alkyl having up to 5 carbon atoms which is optionally substituted by hydroxyl,

or

$R^3$ and $R^4$ together with the nitrogen atom form a 5- to 7-membered unsaturated or saturated or partially unsaturated optionally benzo-fused heterocycle which may optionally contain up to 3 heteroatoms from the group consisting of S, N, O or a radical of the formula $-NR^{37}$,

in which

$R^{37}$ represents hydrogen, hydroxyl, formyl, trifluoromethyl, straight-chain or branched acyl, alkoxy or alkoxycarbonyl having in each case up to 4 carbon atoms,
or represents straight-chain or branched alkyl having up to 6 carbon atoms which is optionally mono- to polysubstituted by identical or different substituents from the group consisting of hydroxyl, trifluoromethyl, carboxyl, straight-chain or branched alkoxy or alkoxycarbonyl having in each case up to 6 carbon atoms or by groups of the formula $-(D)_f-NR^{38}R^{39}$, $-CO-(CH_2)_g-O-CO-R^{40}$, $-CO-(CH_2)_h-OR^{41}$ or $-P(O)(OR^{42})(OR^{43})$,
in which

g and h are identical or different and represent a number 1, 2, 3 or 4,

and

f represents a number 0 or 1,

D represents a group of the formula -CO or $-SO_2$,

$R^{38}$ and $R^{39}$ are identical or different and have the meaning of $R^7$ and $R^8$ given above,

$R^{40}$ represents straight-chain or branched alkyl having up to 6 carbon atoms,

$R^{41}$ represents straight-chain or branched alkyl having up to 6 carbon atoms,

$R^{42}$ and $R^{43}$ are identical or different and represent hydrogen or straight-chain or branched alkyl having up to 4 carbon atoms,

or

$R^{37}$ represents a radical of the formula $-(CO)_i-E$,
in which

i represents a number 0 or 1,

E represents cycloalkyl having 3 to 7 carbon atoms or benzyl, represents aryl having 6 to 10 carbon atoms or a 5- to 6-membered aromatic heterocycle having up to 4 heteroatoms from the group consisting of S, N and O, where the ring systems listed above are optionally mono- to polysubstituted by identical or different substituents from the group consisting of nitro, halogen, $-SO_3H$, straight-chain or branched alkoxy having up to 6 carbon atoms, hydroxyl, trifluoromethyl, trifluoromethoxy or by a radical of the formula $-SO_2-NR^{44}R^{45}$,
in which

$R^{44}$ and $R^{45}$ have the meaning of $R^{18}$ and $R^{19}$ given above and are identical to or different from this meaning,

or

E represents radicals of the formulae

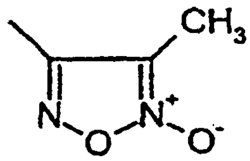

,

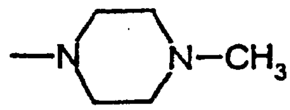

or

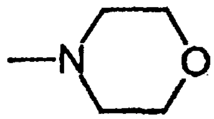

,

and the heterocycle listed under $R^3$ and $R^4$, which is formed together with the nitrogen atom, is optionally mono- to polysubstituted by identical or different substituents, if appropriate also geminally, by hydroxyl, formyl, carboxyl, straight-chain or branched acyl or alkoxycarbonyl having in each case up to 6 carbon atoms, nitro and groups of the formulae $-P(O)(OR^{46})(OR^{47})$,

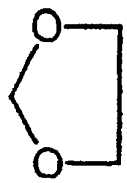

,

$=NR^{48}$ or $-(CO)_jNR^{49}R^{50}$,

in which

$R^{46}$ and $R^{47}$ have the meaning of $R^{10}$ and $R^{11}$ given above and are identical to or different from this meaning,

$R^{48}$ is hydroxyl or straight-chain or branched alkoxy having up to 4 carbon atoms,

j is a number 0 or 1,
and

$R^{49}$ and $R^{50}$ are identical or different and have the meaning of $R^{14}$ and $R^{15}$ given above,

and/or the heterocycle listed under $R^3$ and $R^4$, which is formed together with the nitrogen atom, is optionally substituted by straight-chain or branched alkyl having up to 6 carbon atoms which is optionally mono- to polysubstituted by identical or different substituents from the group consisting of hydroxyl, halogen, carboxyl, cycloalkyl or cycloalkyloxy having in each case 3 to 8 carbon atoms, straight-chain or branched alkoxy or alkoxycarbonyl having in each case up to 6 carbon atoms or by a radical of the formula $-SO_3H$, $-NR^{51}R^{52}$ or $P(O)OR^{53}OR^{54}$,
in which

$R^{51}$ and $R^{52}$ are identical or different and represent hydrogen, phenyl, carboxyl, benzyl or straight-chain or branched alkyl or alkoxy having in each case up to 6 carbon atoms,

$R^{53}$ and $R^{54}$ are identical or different and have the meaning of $R^{10}$ and $R^{11}$ given above,

and/or the alkyl is optionally substituted by aryl having 6 to 10 carbon atoms which for its part may be mono- to polysubstituted by identical or different substituents from the group consisting of halogen, hydroxyl, straight-chain or branched alkoxy having up to 6 carbon atoms, or by a group of the formula $-NR^{51'}R^{52'}$,
in which

$R^{51'}$ and $R^{52'}$ have the meaning of $R^{51}$ and $R^{52}$ given above and are identical to or different from this meaning,

and/or the heterocycle listed under $R^3$ and $R^4$, which is formed together with the nitrogen atom, is optionally substituted by aryl having 6 to 10 carbon atoms or by a 5- to 7-membered saturated, partially unsaturated or unsaturated heterocycle having up to 3 heteroatoms from the group consisting of S, N and O, if appropriate also attached via an N-function, where the ring systems for their part may be substituted by hydroxyl or by straight-chain or branched alkyl or alkoxy having in each case up to 6 carbon atoms,
or

$R^3$ and $R^4$ together with the nitrogen atom form radicals of the formulae

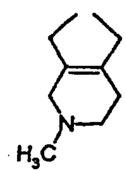

,

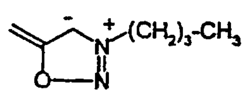

,

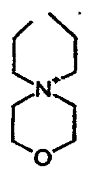

or

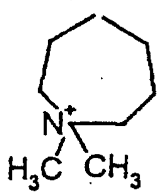

,

$R^5$ and $R^6$ are identical or different and represent hydrogen, straight-chain or branched alkyl having up to 6 carbon atoms, hydroxyl or represent straight-chain or branched alkoxy having up to 6 carbon atoms.

and their respective salts, hydrates, alkoxides and tautomers.

**13.** Combination preparation according to any of Claims 1 to 10, **characterized in that** the PDE V inhibitor (active compound component A) is selected from the group consisting of (a) 5-[2-ethoxy-5-(4-methyl-1-piperazinylsulfonyl)-phenyl]-1-methyl-3-n-propyl-1,6-dihydro-7H-pyrazolo-[4,3-d]-pyrimidin-7-one (sildenafil) and its salts, hydrates, alkoxides and tautomers; and (b) 2-[2-ethoxy-5-(4-ethyl-piperazine-1-sulfonyl)-phenyl]-5-methyl-7-propyl-3H-imidazo[5,1-f]-[1,2,4]-triazin-4-one and its salts, hydrates, alkoxides and tautomers.

**14.** Combination preparation according to Claim 13, in that the PDE V inhibitor (active compound component A) is 5-[2-ethoxy-5-(4-methyl-1-piperazinylsulfonyl)-phenyl]-1-methyl-3-n-propyl-1,6-dihydro-7H-pyrazolo-[4,3-d]-pyrimidin-7-one citrate (sildenafil citrate, Viagra™) or 2-[2-ethoxy-5-(4-ethylpiperazine-1-sulfonyl)-phenyl]-5-methyl-7-propyl-3H-imidazo[5,1-f][1,2,4]triazin-4-one hydrochloride trihydrate.

**15.** Use of antilipemics for enhancing the activity of PDE V inhibitors.

**16.** Use according to Claim 15 for manufacturing a medicament for the treatment of sexual dysfunction in men and women, in particular in the treatment of erectile dysfunction.

**17.** Use according to Claim 15 or 16, **characterized in that** the antilipemic and the PDE V inhibitor are used either simultaneously or else successively.

**18.** Use according to any of Claims 15 to 17, **characterized in that** the antilipemic and the PDE V inhibitor are present as a functional unit, in particular in the form of a mixture, a mix or a blend.

**19.** Use according to any of Claims 15 to 17, **characterized in that** the antilipemic and the PDE V inhibitor are present (spatially) separated, in particular as a kit-of-parts.

**20.** Use according to any of Claims 15 to 19, **characterized in that** the antilipemic is selected from the compounds defined in Claims 7 to 10.

**21.** Use according to any of Claims 15 to 20, **characterized in that** the PDE V inhibitor is selected from the compounds defined in Claims 11 to 14.

## Revendications

**1.** Combinaison pharmaceutique, contenant comme constituants doués d'activité pharmaceutique au moins un composant actif A et au moins un composant actif B, **caractérisée en ce que en ce que** le composant actif A est un inhibiteur de PDE-V et le composant actif B est un hypolipidémiant.

**2.** Combinaison pharmaceutique suivant la revendication 1, destinée au traitement de la dysfonction sexuelle chez l'homme ou la femme.

**3.** Combinaison pharmaceutique suivant la revendication 2, destinée au traitement de la dysfonction de l'érection.

**4.** Combinaison pharmaceutique suivant l'une des revendications 1 à 3, **caractérisée en ce que** les deux composants actifs A et B sont utilisés simultanément ou bien avec échelonnement dans le temps.

**5.** Combinaison pharmaceutique suivant l'une des revendications 1 à 4, **caractérisée en ce que** les composants actifs A et B sont présents comme unité fonctionnelle, en particulier sous forme d'un mélange, d'une mixture ou d'un agrégat.

**6.** Combinaison pharmaceutique suivant l'une des revendications 1 à 4, **caractérisée en ce que** les composants actifs A et B sont présents séparément l'un de l'autre (dans l'espace), en particulier comme "kit-of-parts".

**7.** Combinaison pharmaceutique suivant l'une des revendications 1 à 6, **caractérisée en ce que** l'hypolipidémiant (composant actif B) est choisi dans le groupe (a) des inhibiteurs de HMG-CoA-réductase ; (b) des inhibiteurs de squalène-synthétase ; (c) des inhibiteurs d'absorption des acides biliaires ("Bile acid sequestrants") ; (d) l'acide fibrique et ses dérivés ; (e) l'acide nicotinique et ses analogues ; (f) les $\bar{\omega}$3-acides gras.

**8.** Combinaison pharmaceutique suivant la revendication 7, **caractérisée en ce que** l'hypolipidémiant (composant actif B) est un inhibiteur de HMG-CoA-réductase et est choisi en particulier dans le groupe des statines, avantageusement dans le groupe de l'atorvastatine, de la cérivastatine, de la fluvastatine, de la lovastatine, de la pravastatine, de l'itavastatine, de la simvastatine et de l'acide (+)-(3R,5S)-bis-(7-(4-(4-fluorophényl)-6-isopropyl-2-(N-méthyl-N-méthanesulfonylamino)-pyrimidine-5-yl)-3,5-dihydroxy-6(E)-hepténoïque ainsi leurs sels, hydrates, alcoolates, esters et tautomères correspondants.

**9.** Combinaison pharmaceutique suivant la revendication 8, **caractérisée en ce que** l'hypolipidémiant (composant actif B) est l'atorvastatine ou un sel, hydrate, alcoolate, ester et tautomère de ce composé.

**10.** Combinaison pharmaceutique suivant la revendication 8, **caractérisée en ce que** l'hypolipidémiant (composant actif B) est la cérivastatine ou un sel, hydrate, alcoolate, ester et tautomère de ce composé.

**11.** Combinaison pharmaceutique suivant l'une des revendications 1 à 10, **caractérisée en ce que** l'inhibiteur de PDE-V (composant actif A) est choisi dans le groupe des pyrazolopyrimidones de formule générale suivante

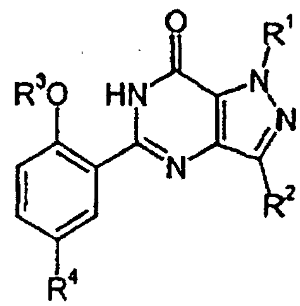

dans laquelle

| | |
|---|---|
| $R^1$ | représente l'hydrogène, un reste alkyle en $C_1$ à $C_3$ ; perfluoralkyle en $C_1$ à $C_3$ ; ou cycloalkyle en $C_3$ à $C_5$ ; |
| $R^2$ | représente l'hydrogène, un reste alkyle en $C_1$ à $C_6$, éventuellement substitué avec un radical cycloalkyle en $C_3$ à $C_6$ ; un reste perfluoralkyle en $C_1$ à $C_3$ ; ou un reste cycloalkyle en $C_3$ à $C_6$ ; |
| $R^3$ | représente un reste alkyle en $C_1$ à $C_6$, éventuellement substitué avec un radical cycloalkyle en $C_3$ -à $C_6$ ; un reste perfluoralkyle en $C_1$ à $C_6$, cycloalkyle en $C_3$ à $C_5$ ; un reste alcényle en $C_3$ à $C_6$ ; ou un reste alcynyle en $C_3$ à $C_6$ ; |
| $R^4$ | est un reste alkyle en $C_1$ à $C_4$, éventuellement substitué avec un radical OH, $NR^5R^6$, CN, $CONR^5R^6$ ou $CO_2R^7$ ; un reste alcényle en $C_2$ à $C_4$, éventuellement substitué avec un radical CN, $CONR^5R^6$ ou $CO_2R^7$ ; un reste alcanoyle en $C_2$ à $C_4$, éventuellement substitué avec un radical $NR^5R^6$ ; un reste (hydroxy)-(alkyle en $C_2$ à $C_4$), éventuellement substitué avec un radical $NR^5R^6$, un reste (alkoxy en $C_2$ ou $C_3$)-(alkyle en $C_1$ ou $C_2$), éventuellement substitué avec un radical OH ou $NR^5R^6$, $CO_2R^7$ ; un halogène ; un groupe $NR^5R^6$, $NHSO_2NR^5R^6$ ; $NHSO_2R^8$ ; $SO_2NR^9R^{10}$ ; ou un groupe phényle, pyridyle, pyrimidinyle, imidazolyle, oxazolyle, thiazolyle, thiényle ou thiazolyle, chacun d'eux étant éventuellement substitué avec un radical méthyle ; |
| $R^5$ et $R^6$ | représentent chacun, indépendamment, l'hydrogène ou un reste alkyle en $C_1$ à $C_4$ ; ou forment conjointement avec l'atome d'azote auquel ils sont liés un groupe pyrrolidinyle, pipéridino, morpholino, 4-N($R^{11}$)pipérazinyle ou imidazolyle, ce groupe étant éventuellement substitué avec un radical méthyle ou OH ; |
| $R^7$ | représente l'hydrogène ou un reste alkyle en $C_1$ à $C_4$ ; |
| $R^8$ | représente un reste alkyle en $C_1$ à $C_3$, éventuellement substitué avec un radical $NR^5R^6$ ; |
| $R^9$ et $R^{10}$ | forment, conjointement avec l'atome d'azote auquel ils sont liés, un groupe pyrrolidinyle, pipéridino, morpholino, 4-N($R^{12}$)pipérazinyle, ce groupe étant éventuellement substitué avec un radical alkyle en $C_1$ à $C_4$, alkoxy en $C_1$ à $C_3$, $NR^{13}R^{14}$ ou $CONR^{13}R^{14}$ ; |
| $R^{11}$ | représente l'hydrogène, un reste alkyle en $C_1$ à $C_3$, éventuellement substitué avec un radical phényle ; un reste (hydroxy)-(alkyle en $C_2$ ou $C_3$) ; ou alcanoyle en $C_1$ à $C_4$ ; |
| $R^{12}$ | représente l'hydrogène, un reste alkyle en $C_1$ à $C_6$, (alkoxy en $C_1$ à $C_3$)-(alkyle en $C_2$ à $C_6$) ; (hydroxy) -(alkyle en $C_2$ à $C_6$) ; ($R^{13}R^{14}$N)- (alkyle en $C_2$ à $C_6$) ; ($R^{13}R^{14}$NOC) - (alkyle en $C_1$ à $C_6$) ; $CONR^{13}R^{14}$ ; $CSNR^{13}R^{14}$ ou C(NH) $NR^{13}R^{19}$ ; et |
| $R^{13}$ et $R^{14}$ | représentent chacun, indépendamment, l'hydrogène ; un reste alkyle en $C_1$ à $C_4$ ; (alkoxy en $C_1$ à $C_3$)-(alkyle en $C_2$ à $C_4$) ; ou (hydroxy)-(alkyle en $C_2$ à $C_4$), |

ainsi que leurs sels, hydrates, alcoolates et tautomères correspondants.

**12.** Combinaison pharmaceutique suivant l'une des revendications 1 à 10, **caractérisée en ce que** l'inhibiteur de PDE-V (composant actif A) est choisi dans le groupe des imidazotriazinones à substituant 2-phényle, de formule générale

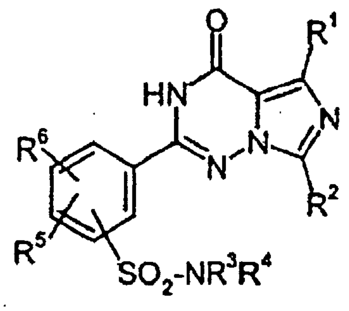

(I)

dans laquelle

$R^1$ est l'hydrogène ou un reste alkyle linéaire ou ramifié ayant jusqu'à 4 atomes de carbone ;

$R^2$ est un reste alkyle linéaire ayant jusqu'à 4 atomes de carbone ;

$R^3$ et $R^4$ sont identiques ou différents et représentent l'hydrogène ou un reste alcényle ou alkoxy linéaire ou ramifié ayant chacun jusqu'à 8 atomes de carbone, ou bien

une chaîne alkylique linéaire ou ramifiée ayant jusqu'à 10 atomes de carbone, qui est éventuellement interrompue par un atome d'oxygène et qui est éventuellement substituée une ou plusieurs fois, identiques ou différentes, par un radical trifluorométhyle, trifluorométhoxy, hydroxy, halogéno, carboxyle, benzyloxycarbonyle, alkoxycarbonyle linéaire ou ramifié ayant jusqu'à 6 atomes de carbone et/ou par des restes de formules $-SO_3H$, $-(A)_a-NR^7R^8$, $-O-CO-NR^{7'}R^{8'}$, $-S-(O)_b-R^9$, $-P(O)(OR^{10})(OR^{11})$,

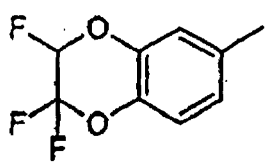

,

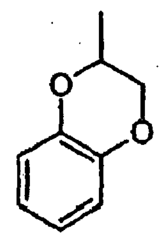

,

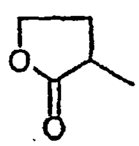

et/ou

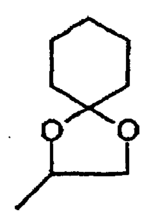

où

a et b sont égaux ou différents et représentent le nombre 0 ou 1,

A est un reste CO ou $SO_2$,

$R^7$, $R^{7'}$, $R^8$ et $R^{8'}$ sont identiques ou différents et représentent l'hydrogène,

ou bien

un reste cycloalkyle ayant 3 à 8 atomes de carbone, aryle ayant 6 à 10 atomes de carbone, un hétérocycle pentagonal ou hexagonal insaturé, partiellement insaturé ou saturé, éventuellement condensé au benzène et ayant jusqu'à 3 hétéroatomes de la série S, N et/ou O, les systèmes de noyaux indiqués ci-dessus étant éventuellement substitués une ou plusieurs fois identiques ou différentes par un radical hydroxy, nitro, trifluorométhyle, trifluorométhoxy, carboxyle, halogéno, alkoxy ou alkoxycarbonyle linéaire ou ramifié ayant chacun jusqu'à 6 atomes de carbone ou par un groupe de formule-$(SO_2)_c$-$NR^{12}$ $R^{13}$,
où

c représente le nombre 0 ou 1;
$R^{12}$ et $R^{13}$ sont identiques ou différents et représentent l'hydrogène ou un reste alkyle linéaire ou ramifié ayant jusqu'à 5 atomes de carbone,

ou bien
$R^7$, $R^{7'}$, $R^8$ et $R^{8'}$ représentent un groupe alkoxy linéaire ou ramifié ayant jusqu'à 6 atomes de carbone, ou bien un reste alkyle linéaire ou ramifié ayant jusqu'à 8 atomes de carbone, qui est éventuellement substitué une ou plusieurs fois identiques ou différentes par un radical hydroxy, halogéno, aryle ayant 6 à 10 atomes de carbone, alkoxy ou alkoxycarbonyle linéaire ou ramifié ayant chacun jusqu'à 6 atomes de carbone ou par un groupe de formule - $(CO)_d$-$NR^{14}R^{15}$,
formule dans laquelle

$R^{14}$ et $R^{15}$ sont identiques ou différents et représentent l'hydrogène ou un reste alkyle linéaire ou ramifié ayant jusqu'à 4 atomes de carbone,

et

d représente le nombre 0 ou 1,

ou
$R^7$ et $R^8$ et/ou $R^{7'}$ et $R^{8'}$ forment, conjointement avec l'atome d'azote, un hétérocycle saturé pentagonal à heptagonal qui peut éventuellement contenir encore un autre hétéroatome de la série S ou O ou un reste de formule -$NR^{16}$,
où

$R^{16}$ représente l'hydrogène, un reste aryle ayant 6 à 10 atomes de carbone, un reste benzyle, un hétérocycle pentagonal à heptagonal aromatique ou saturé ayant jusqu'à 3 hétéroatomes de la série S, N et/ou O, qui est éventuellement substitué par un radical méthyle,
ou bien
un reste alkyle linéaire ou ramifié ayant jusqu'à 6 atomes de carbone, qui est éventuellement substitué par un radical hydroxy,

$R^9$ est un reste aryle ayant 6 à 10 atomes de carbone, ou un reste alkyle linéaire ou ramifié ayant jusqu'à 4 atomes de carbone,
$R^{10}$ et $R^{11}$ sont identiques ou différents et représentent l'hydrogène ou un reste alkyle linéaire ou ramifié ayant jusqu'à 4 atomes de carbone,

et/ou la chaîne alkylique indiquée ci-dessus pour $R^3$/$R^4$ est éventuellement.substituée par un radical cycloalkyle ayant 3 à 8 atomes de carbone, aryle ayant 6 à 10 atomes de carbone ou par un hétérocycle pentagonal à heptagonal partiellement insaturé, saturé ou insaturé, éventuellement condensé au benzène, qui peut contenir jusqu'à 4 hétéroatomes de la série S, N, O ou un reste de formule -$NR^{17}$, où

$R^{17}$ représente l'hydrogène, un groupe hydroxy, formyle, trifluorométhyle, un reste acyle ou alkoxy linéaire ou ramifié ayant chacun jusqu'à 4 atomes de carbone,
ou un reste alkyle linéaire ou ramifié ayant jusqu'à 6 atomes de carbone, qui est éventuellement substitué une ou plusieurs fois identiques ou différentes par un radical hydroxy ou par un radical alkoxy linéaire ou ramifié ayant jusqu'à 6 atomes de carbone,

et le reste aryle et l'hétérocycle sont éventuellement substitués une ou plusieurs fois identiques ou différentes par un radical nitro, halogéno, -$SO_3H$, alkyle ou alkoxy linéaire ou ramifié ayant chacun jusqu'à 6 atomes de carbone, hydroxy, trifluorométhyle, trifluorométhoxy et/ou par un reste de formule -$SO_2NR^{18}R^{19}$,
où

$R^{18}$ et $R^{19}$ sont identiques ou différents et représentent l'hydrogène ou un reste alkyle linéaire ou ramifié ayant jusqu'à 6 atomes de carbone,

et/ou

$R^3$ et $R^4$ représentent un groupe de formule $-NR^{20}R^{21}$,

dans laquelle

$R^{20}$ et $R^{21}$ ont la définition indiquée ci-dessus pour $R^{18}$ et $R^{19}$ et y sont identiques ou en sont différents,

et/ou

$R^3$ ou $R^4$ représentent un reste adamantyle, ou bien

des restes de formules

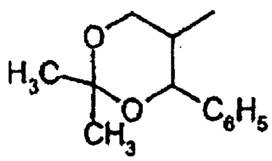
,
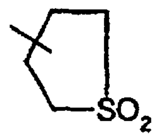
,
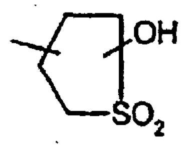

ou

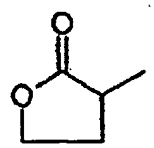

ou un reste cycloalkyle ayant 3 à 8 atomes de carbone, aryle ayant 6 à 10 atomes de carbone ou un hétérocycle pentagonal à heptagonal partiellement insaturé, saturé ou insaturé, éventuellement condensé au benzène, qui peut contenir jusqu'à 4 hétéroatomes de la série S, N ou O ou un reste de formule $-NR^{22}$,
où

$R^{22}$ a la définition indiquée ci-dessus pour $R^{16}$ et y est identique ou en est différent, ou bien un reste carboxyle, formyle ou un reste acyle linéaire ou ramifié ayant jusqu'à 5 atomes de carbone, et les restes cycloalkyle, aryle et/ou l'hétérocycle étant éventuellement substitués une ou plusieurs fois identiques ou différentes par un radical halogéno, triazolyle, trifluorométhyle, trifluorométhoxy, carboxyle, acyle ou alkoxycarbonyle linéaire ou ramifié ayant chacun jusqu'à 6 atomes de carbone, nitro, et/ou par des groupes de formules $-SO_3H$, $-OR^{23}$, $(SO_2)_eNR^{24}R^{25}$, $-P(O)(OR^{26})(OR^{27})$
où

e représente le nombre 0 ou 1

$R^{23}$ est un reste de formule

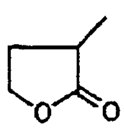

ou

un reste cycloalkyle ayant 3 à 7 atomes de carbone ou l'hydrogène ou un reste alkyle linéaire ou ramifié ayant jusqu'à 4 atomes de carbone, éventuellement substitué par un radical cycloalkyle ayant 3 à 7 atomes de carbone, benzyloxy, tétrahydropyrannyle, tétrahydrofurannyle, alkoxy ou alkoxycarbonyle linéaire ou ramifié ayant chacun jusqu'à 6 atomes de carbone, carboxyle, benzyloxycarbonyle, ou phényle, qui peut lui-même être substitué une ou plusieurs fois identiques ou différentes par un radical alkoxy linéaire ou ramifié ayant jusqu'à 4 atomes de carbone, hydroxy ou halogéno, et/ou le reste alkyle est éventuellement substitué par des restes de formules -CO-$NR^{28}R^{29}$ ou -CO-$R^{30}$,
où

$R^{28}$ et $R^{29}$ sont identiques ou différents et représentent l'hydrogène ou un reste alkyle linéaire ou ramifié ayant jusqu'à 8 atomes de carbone, ou bien
$R^{28}$ et $R^{29}$ forment conjointement avec l'atome d'azote un hétérocycle saturé pentagonal à heptagonal, qui peut éventuellement contenir un autre hétéroatome de la série S ou O, et
$R^{30}$ est un reste phényle ou adamantyle,

$R^{24}$ et $R^{25}$ ont la définition indiquée ci-dessus pour $R^{18}$ et $R^{19}$ et y sont identiques ou en sont différents,
$R^{26}$ et $R^{27}$ ont la définition indiquée ci-dessus pour $R^{10}$ et $R^{11}$ et y sont identiques ou en sont différents,

et/ou le reste cycloalkyle, le reste aryle et/ou l'hétérocycle sont éventuellement substitués par un radical alkyle linéaire ou ramifié ayant jusqu'à 6 atomes de carbone, qui est éventuellement substitué par un radical hydroxy, carboxyle, par un hétérocycle pentagonal à heptagonal ayant jusqu'à 3 hétéroatomes de la série S, N et/ou O ou par des groupes de formule -$SO_2$-$R^{31}$, P (O) ($OR^{32}$) ($OR^{33}$) ou -$NR^{34}R^{35}$,
où

$R^{31}$ représente l'hydrogène ou a la définition indiquée ci-dessus pour $R^9$ et y est identique ou en est différent,
$R^{32}$ et $R^{33}$ ont la définition indiquée ci-dessus pour $R^{10}$ et $R^{11}$ et y sont identiques ou en sont différents,
$R^{34}$ et $R^{35}$ sont identiques ou différents et représentent l'hydrogène ou un reste alkyle linéaire ou ramifié ayant jusqu'à 6 atomes de carbone, qui est éventuellement substitué par un radical hydroxy ou un radical alkoxy linéaire ou ramifié ayant jusqu'à 4 atomes de carbone, ou
$R^{34}$ et $R^{35}$ forment, conjointement avec l'atome d'azote, un hétérocycle pentagonal ou hexagonal saturé, qui peut contenir un autre hétéroatome de la série S ou O ou un reste de formule -$NR^{36}$,
où

$R^{36}$ représente l'hydrogène, un reste hydroxy, alkoxycarbonyle linéaire ou ramifié ayant jusqu'à 7 atomes de carbone ou alkyle linéaire ou ramifié ayant jusqu'à 5 atomes de carbone, qui est éventuellement substitué par un radical hydroxy,

ou bien

$R^3$ et $R^4$ forment, conjointement avec l'atome d'azote, un hétérocycle pentagonal à heptagonal non saturé ou saturé ou partiellement insaturé, éventuellement condensé au benzène, qui peut éventuellement contenir jusqu'à 3 hétéroatomes de la série S, N, O ou un reste de formule -$NR^{37}$,

où

$R^{37}$ représente l'hydrogène, un groupe hydroxy, formyle, trifluorométhyle, un reste acyle, alkoxy ou alkoxycarbonyle linéaire ou ramifié ayant chacun jusqu'à 4 atomes de carbone,
ou un reste alkyle linéaire ou ramifié ayant jusqu'à 6 atomes de carbone, qui est éventuellement substitué une ou plusieurs fois identiques ou différentes par un radical hydroxy, trifluorométhyle, carboxyle, alkoxy ou alkoxycarbonyle linéaire ou ramifié ayant chacun jusqu'à 6 atomes de carbone ou par des groupes de formule -(D)f-$NR^{38}R^{39}$, -CO-$(CH_2)_g$-O-CO-$R^{40}$, -CO-$(CH_2)_h$-$OR^{41}$ ou -P (O) ($OR^{42}$) ($OR^{43}$),
où
g et h sont égaux ou différents et représentent le nombre 1, 2, 3 ou 4;
et

f représente le nombre 0 ou 1,
D est un groupe de formule -CO ou $-SO_2$,
$R^{38}$ et $R^{39}$ sont identiques ou différents et ont la définition indiquée ci-dessus pour $R^7$ et $R^8$,
$R^{40}$ est un reste alkyle linéaire ou ramifié ayant jusqu'à 6 atomes de carbone,
$R^{41}$ est un reste alkyle linéaire ou ramifié ayant jusqu'à 6 atomes de carbone,
$R^{42}$ et $R^{43}$ sont identiques ou différents et représentent l'hydrogène ou un reste alkyle linéaire ou ramifié ayant jusqu'à 4 atomes de carbone,

ou bien

$R^{37}$ représente un reste de formule $-(CO)_i-E$, dans laquelle

i représente le nombre 0 ou 1,
E est un reste cycloalkyle ayant 3 à 7 atomes de carbone ou un reste benzyle, un reste acyle ayant 6 à 10 atomes de carbone ou un hétérocycle aromatique pentagonal ou hexagonal ayant jusqu'à 4 hétéroatomes de la série S, N et/ou O, les systèmes de noyaux indiqués ci-dessus étant éventuellement substitués une ou plusieurs fois identiques ou différentes par un radical nitro, halogéno, $-SO_3H$, alkoxy linéaire ou ramifié ayant jusqu'à 6 atomes de carbone, hydroxy, trifluorométhyle, trifluorométhoxy ou par un reste de formule $-SO_2-NR^{44}R^{45}$,
où

$R^{44}$ et $R^{45}$ ont la définition indiquée ci-dessus pour $R^{18}$ et $R^{19}$ et y sont identiques ou en sont différents,

ou bien

E représente des restes de formules

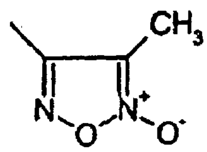 , 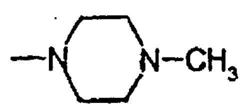

ou

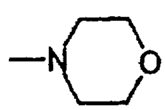

et l'hétérocycle indiqué pour $R^3$ et $R^4$, formé conjointement avec l'atome d'azote, est éventuellement substitué une ou plusieurs fois identiques ou différentes, éventuellement aussi de façon géminée, par un radical hydroxy, formyle, carboxyle, un radical acyle ou alkoxycarbonyle linéaire ou ramifié ayant chacun jusqu'à 6 atomes de carbone, un radical nitro et des groupes de formules $-P(O)(OR^{46})(OR^{47})$,

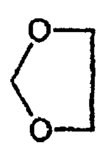 ,

$=NR^{48}$ ou $-(CO)_jNR^{49}R^{50}$

où

$R^{46}$ et $R^{47}$ ont la définition indiquée ci-dessus pour $R^{10}$ et $R^{11}$ et y sont identiques ou en sont différents,

$R^{48}$ est un groupe hydroxy ou un reste alkoxy linéaire ou ramifié ayant jusqu'à 4 atomes de carbone,

j représente le nombre 0 ou 1,

et

$R^{49}$ et $R^{50}$ sont identiques ou différents et ont la définition indiquée ci-dessus pour $R^{14}$ et $R^{15}$,

et/ou l'hétérocycle indiqué pour $R^3$ et $R^4$, formé conjointement avec l'atome d'azote, est éventuellement substitué par un radical alkyle linéaire ou ramifié ayant jusqu'à 6 atomes de carbone, qui est éventuellement substitué une ou plusieurs fois identiques ou différentes par un radical hydroxy, halogéno, carboxyle, un radical cycloalkyle ou cycloalkyloxy ayant chacun 3 à 8 atomes de carbone, un radical alkoxy ou alkoxycarbonyle linéaire ou ramifié ayant chacun jusqu'à 6 atomes de carbone ou par un reste de formule $-SO_3H$, $-NR^{51}R^{52}$ ou $P(O)OR^{53}OR^{54}$,
où

$R^{51}$ et $R^{52}$ sont identiques ou différents et représentent l'hydrogène, un reste phényle, carboxyle, benzyle ou un reste alkyle ou alkoxy linéaire ou ramifié ayant chacun jusqu'à 6 atomes de carbone,

$R^{53}$ et $R^{54}$ sont identiques ou différents et ont la définition indiquée ci-dessus pour $R^{10}$ et $R^{11}$, et/ou le reste alkyle est éventuellement substitué par un radical aryle ayant 6 à 10 atomes de carbone, qui peut lui-même être substitué une ou plusieurs fois identiques ou différentes par un radical halogéno, hydroxy, alkoxy linéaire ou ramifié ayant jusqu'à 6 atomes de carbone ou par un groupe de formule $-NR^{51'}R^{52'}$,

où

$R^{51'}$ et $R^{52'}$ ont la définition indiquée ci-dessus pour $R^{51}$ et $R^{52}$ et y sont identiques ou en sont différents,

et/ou l'hétérocycle indiqué pour $R^3$ et $R^4$, formé conjointement avec l'atome d'azote, est éventuellement substitué par un reste aryle ayant 6 à 10 atomes de carbone ou par un hétérocycle pentagonal à heptagonal saturé, partiellement insaturé ou insaturé ayant jusqu'à 6 hétéroatomes de la série S, N et/ou O, éventuellement relié aussi par l'intermédiaire d'une fonction N, les systèmes de noyaux pouvant eux-mêmes être substitués par un radical hydroxy ou par un reste alkyle ou alkoxy linéaire ou ramifié ayant chacun jusqu'à 6 atomes de carbone,
ou bien
$R^3$ et $R^4$ forment conjointement avec l'atome d'azote des restes de formules

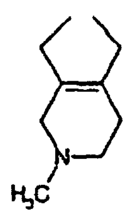
,
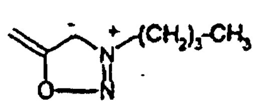
,
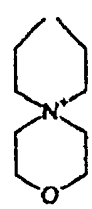

ou

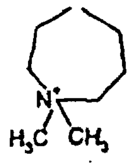

$R^5$ et $R^6$ sont identiques ou différents et représentent l'hydrogène, un reste alkyle linéaire ou ramifié ayant jusqu'à 6 atomes de carbone, un reste hydroxy ou un reste alkoxy linéaire ou ramifié ayant jusqu'à 6 atomes de carbone,

ainsi que leurs sels, leurs hydrates, leurs alcoolates et leurs tautomères correspondants.

**13.** Combinaison pharmaceutique suivant l'une des revendications 1 à 10, **caractérisée en ce que** l'inhibiteur de PDE-V (composant actif A) est choisi dans le groupe de (a) la 5-[2-éthoxy-5-(4-méthyl-1-pipérazinylsulfonyl)-phényl]-1-méthyl-3-n-propyl-1,6-dihydro-7H-pyrazolo-[4,3-d]-pyrimidine-7-one (sildenafil) ainsi que ses sels, hydrates, alcoolates et tautomères ; et (b) la 2-[2-éthoxy-5-(4-éthylpipérazine-1-sulfonyl)-phényl]-5-méthyl-7-propyl-3H-imidazo[5,1-f]-[1,2,4]-triazine-4-one ainsi que ses sels, hydrates, alcoolates et tautomères.

**14.** Combinaison pharmaceutique suivant la revendication 13, **caractérisée en ce que** l'inhibiteur de PDE-V (composant actif A) est le citrate de 5-[2-éthoxy-5-(4-méthyl-1-pipérazinylsulfonyl)-phényl]-1-méthyl-3-n-propyl-1,6-dihydro-7H-pyrazolo-[4,3-d]-pyrimidine-3-one (citrate de sildenafil, Viagra™) ou le trihydrate du chlorhydrate de 2-[2-éthoxy-5-(4-éthylpipérazine-1-sulfonyl)-phényl]-5-méthyl-7-propyl-3H-imidazo[5,1-f][1,2,4]-triazine-4-one.

**15.** Utilisation d'hypolipidémiants pour accroître l'activité d'inhibiteurs de PDE-V.

**16.** Utilisation suivant la revendication 15 pour la préparation d'un médicament destiné au traitement de la dysfonction sexuelle chez l'homme et la femme, en particulier pour le traitement de la dysfonction de l'érection.

**17.** Utilisation suivant la revendication 15 ou 16, **caractérisée en ce que** l'hypolipidémiant et l'inhibiteur de PDE-V sont utilisés simultanément ou avec échelonnement dans le temps.

**18.** Utilisation suivant l'une des revendications 15 à 17, **caractérisée en ce que** l'hypolipidémiant et l'inhibiteur de PDE-V sont présents comme unité fonctionnelle, en particulier sous forme d'un mélange, d'une mixture ou d'un agrégat.

**19.** Utilisation suivant l'une des revendications 15 à 17, **caractérisée en ce que** l'hypolipidémiant et l'inhibiteur de PDE-V sont présents séparément (dans l'espace) l'un de l'autre, en particulier comme "kit-of-parts".

**20.** Utilisation suivant l'une des revendications 15 à 19, **caractérisée en ce que** l'hypolipidémiant est choisi parmi les composés définis dans les revendications 7 à 10.

**21.** Utilisation suivant l'une des revendications 15 à 20, **caractérisée en ce que** l'inhibiteur de PDE-V est choisi parmi les composés définis dans les revendications 11 à 14.

**Fig. 1**

Wirkmechanismus von PDE V-Inhibitoren

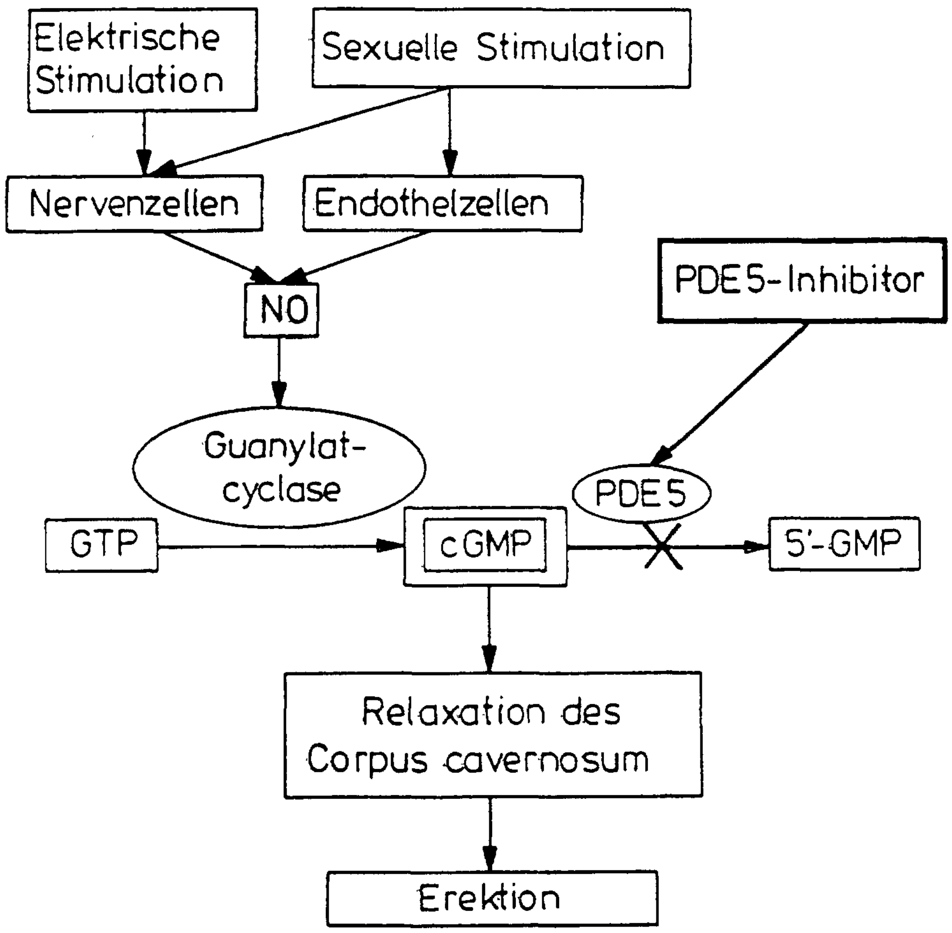

**Fig. 2**

Synergistische Verstärkung der cGMP PDE-inhibitorischen Wirkung